**Europäisches Patentamt**

**European Patent Office**

**Office européen des brevets**

(19)

(11) Publication number: **0 154 539**
**A2**

(12)

# EUROPEAN PATENT APPLICATION

(21) Application number: **85301469.4**

(22) Date of filing: **04.03.85**

(51) Int. Cl.⁴: **C 12 N 15/00**
**C 12 N 1/20**
**//(C12N1/20, C12R1:07, 1:19, 1:465)**

(30) Priority: **06.03.84 US 586592**

(43) Date of publication of application:
**11.09.85 Bulletin 85/37**

(84) Designated Contracting States:
**AT BE CH DE FR GB IT LI LU NL SE**

(71) Applicant: **ELI LILLY AND COMPANY**
**Lilly Corporate Center**
**Indianapolis Indiana 46285(US)**

(72) Inventor: **Schoner, Ronald George**
**274 Larkspur Court**
**Zionsville Indiana 46077(US)**

(72) Inventor: **Schoner, Brigitte Elisabeth**
**274 Larkspur Court** _
**Zionsville Indiana 46077(US)**

(74) Representative: **Hudson, Christopher Mark et al,**
**Erl Wood Manor**
**Windlesham Surrey GU20 6PH(GB)**

(54) Recombinant DNA expression vectors and method for gene expression.

(57) The present invention provides a process for preparing selectable and autonomously replicating recombinant DNA expression vectors which comprise 1) a transcriptional and translational activating sequence which is in the reading frame of a nucleotide sequence that codes for a peptide or polypeptide; 2) a translational stop signal; 3) a translational start signal which is in the reading frame of a nucleotide sequence that codes for a functional polypeptide; and 4) an additional translational stop signal. The above-described nucleotide sequences are engineered to generate, upon transcription, a translatable polycistronic mRNA. The invention further provides a method of use.

**EP 0 154 539 A2**

Croydon Printing Company Ltd

## RECOMBINANT DNA EXPRESSION VECTORS
## AND METHOD FOR GENE EXPRESSION

The present invention relates to a novel selectable and autonomously replicating recombinant DNA expression vector which comprises in series 1) a transcriptional and translational activating sequence which is in the reading frame of a nucleotide sequence that codes for a peptide or polypeptide; 2) a translational stop signal; 3) a translational start signal which is in the reading frame of a nucleotide sequence that codes for a functional polypeptide; and 4) an additional translational stop signal. The above-described nucleotide sequence is engineered to generate, upon transcription, a translatable polycistronic mRNA. The invention further relates to a method of use.

The development and exploitation of recombinant DNA technology has been limited by the general paucity of vectors providing for high levels of gene expresssion. Although the lac (Roberts et al., 1979, Proc. Nat. Acad. Sci. USA 76:5596 and Guarante et al., 1980, Cell 20:543), trp (Hallewell and Emtage, 1980, Gene 9:27), bacteriophage λ P$_L$ (Remaut et al., 1981, Gene 15(1):81, Bernard et al., 1979, Gene 5:59 and Derom et al., 1982, Gene 17:45) and lpp (Ziebel et al., 1981, J. Bacteriol. 145:654, Lee et al., 1981 J. Bacteriol. 146:861 and Nakamura and Inouye, 1979, Cell 18:1109) promoter systems have been incorporated into assorted recombinant DNA vectors, few, if any, other expression systems are available.  In fact, many heterologous genes are not expressed or are, at best, only poorly expressed using these known expression vectors.  Such failure of expression is frequently associated with the transcription of a mRNA that is sub-optimal or incapable of binding to ribosomes.  This may be due to the formation of inappropriate stem and loop structures that sequester sites for ribosome binding, to the general instability of the mRNA transcripts or to the presence of sequences that inhibit ribosome binding or initiation of translation.  In any case, there is a failure of translation and consequently no expression or production of the desired polypeptide.

The present invention solves problems of expression without requiring the synthetic modification of a gene.  Such synthetic modification is not only time consuming and costly but also substantially increases the risk of accidentally introducing errors into a DNA sequence.  This problem is circumvented in the present invention by altering the 5' end of a mRNA transcript without modifying or changing the sequence of a particular gene of interest.

For purposes of the present invention as disclosed and claimed herein, the following terms are as defined below.

Recombinant DNA Cloning Vector - any autonomously replicating agent, including but not limited to plasmids, comprising a DNA molecule to which one or more additional DNA segments can or have been added.

0154539

Recombinant DNA Expression Vector - any recombinant DNA cloning vector into which one or more transcriptional and translational activator sequence(s) have been incorporated.

Transcriptional Activating Sequence - any DNA sequence that directs or provides for the transcription of DNA into a mRNA transcript.

Translational Activating Sequence - any DNA sequence that directs or provides for the translation of a mRNA transcript into a peptide or polypeptide.

Translational Start Signal - any DNA triplet that codes for a translational start codon.

Translational Stop Signal - any DNA triplet that codes for a translational stop codon.

Transformation - the introduction of DNA into a recipient host cell that changes the genotype.

Transformant - a recipient host cell that has undergone transformation.

Restriction Fragment - any linear DNA sequence generated by the action of one or more restricton enzymes.

Functional Polypeptide - a recoverable bioactive heterologous polypeptide or precursor, a recoverable bioactive polypeptide comprising a heterologous polypeptide and a portion or whole of a homologous polypeptide, or a recoverable bioinactive fusion polypeptide comprising a heterologous polypeptide and a bio-inactivating polypeptide which can be specifically cleaved.

:-6354                              -4-

Fused Gene Product - a recoverable heterologous polypeptide which comprises a portion or whole of
a homologous polypeptide.

<u>Description Of The Figures</u>

Figures 1-4 - Schematic illustration of the
construction protocol for plasmid pNM575.

Figures 5-8 - Schematic illustration of the
construction protocol for plasmid pNM789B.

Figure 9     - Restriction site map of plasmids
pCZ114 and pCZ105.1.

Figure 10    - Restriction site map of plasmid
pCZ1140.

Figure 11    - Thymosin alpha 1 synthetic gene.

Figure 12    - Synthesis protocol for nucleotide
fragment T15.

Figure 13    - Construction protocol for plasmid
pThα1.

Figure 14    - Proinsulin synthetic gene.

Figure 15    - Construction protocol for plasmid
pHI7Δ4Δ1.

Figure 16    - Construction protocol for plasmid
pHI104.

The present invention provides a process for preparing a recombinant DNA expression vector which comprises ligating

a)    a transcriptional and translational activating sequence,

b)    a DNA linker comprising a nucleotide sequence that codes for a ribosome binding site and fora a peptide or polypeptide, said peptide or polypeptide coding sequence containing a translational stop signal positioned immediately adjacent and downstream from the nucleotide triplet that codes for the carboxy terminus of said peptide or polypeptide, and a translational start signal which is immediately adjacent and downstream from said stop signal, and

c)    a nucleotide sequence that codes for a functional polypeptide, said functional polypeptide coding sequence containing a translational stop signal positioned immediately adjacent and downstream from the nucleotide triplet that codes for the carboxy terminus of said functional polypeptide,

subject to the limitation that said vector is selectable and autonomously replicating.

The vectors can be constructed by ligating the
XbaaI-HgiAI DNA linker sequence

```
5' CTAGAGGGTATTAATA ATG TTC CCA TTG GAG GAT GAT TAA ATG
    ||||||||||||||| ||| ||| ||| ||| ||| ||| ||| ||| |||
    TCCCATAATTAT TAC AAG GGT AAC CTC CTA CTA ATT TAC

TTC CCA GCC ATG TCC TTG TCC GGC CTG TTT GCC AAC GCT
||| ||| ||| ||| ||| ||| ||| ||| ||| ||| ||| ||| |||
AAG GGT CGG TAC AGG AAC AGG CCG GAC AAA CGG TTG CGA

GTGCT 3'
  |
  C     5'
```

wherein

> A is deoxyadenyl,
> G is deoxyguanyl,
> C is deoxycytosyl and
> T is thymidyl, into the ~10.2 kb BamHI-XbaI and ~0.6 kb BamHi-HgiAI
fragments of plasmid pCZ101.  The resultant plasmid,
designated as plasmid pCZ114, sequentially contains 1)
the E. coli lipoprotein gene transcriptional and translational activating sequence (Nakamura and Inouye, 1979)
in the reading frame of a sequence of nucleotides that
codes for both a ribosome binding site and a peptide of
the sequence MET PHE PRO LEU GLU ASP ASP, wherein

> MET is methionine,
> PHE is phenylalanine,
> PRO is proline,
> LEU is leucine,
> GLU is glutamic acid and
> ASP is aspartic acid;

2) a translational stop signal appropriately positioned and in the reading frame of the aforementioned peptide coding sequence; 3) a translational start signal which is immediately adjacent and downstream from the aforementioned stop signal and which is in the reading frame of a nucleotide sequence coding for methionyl-bovine growth hormone (met-bGH); and 4) a translational stop signal appropriately positioned and in the reading frame of the met-bGH coding sequence.

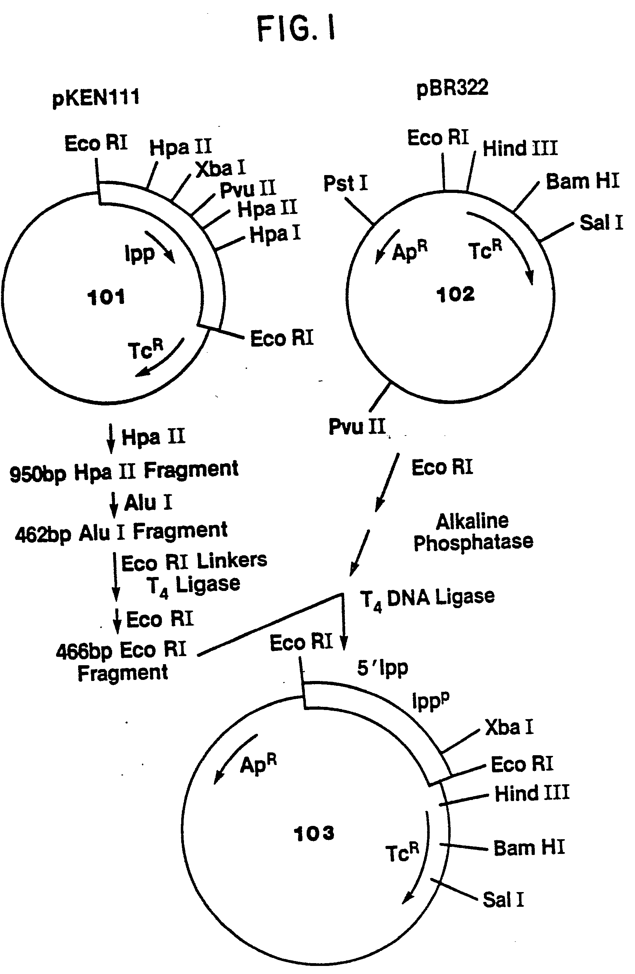

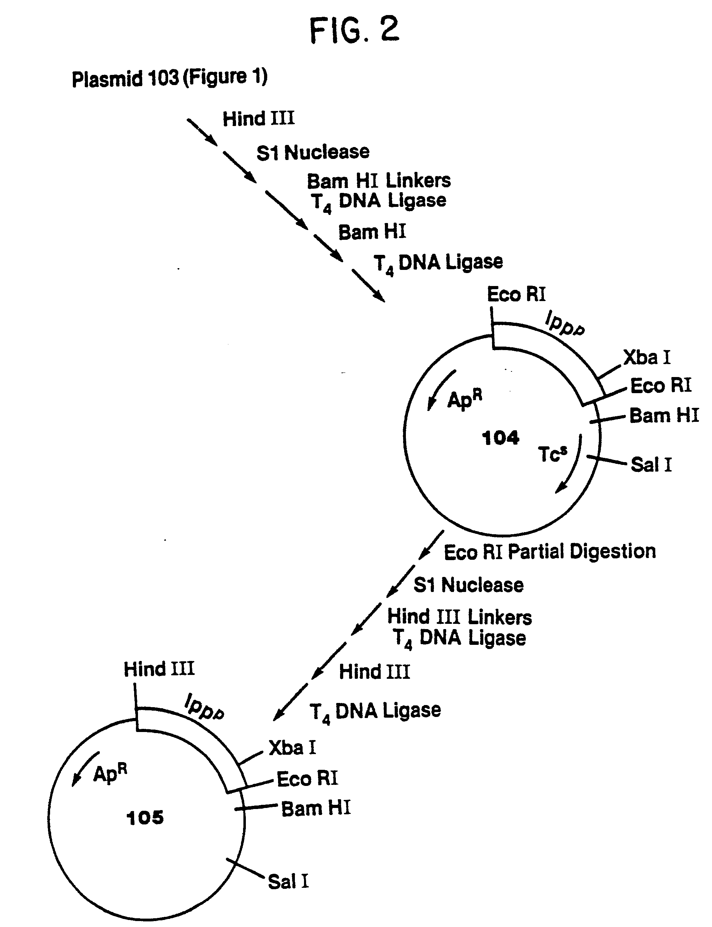

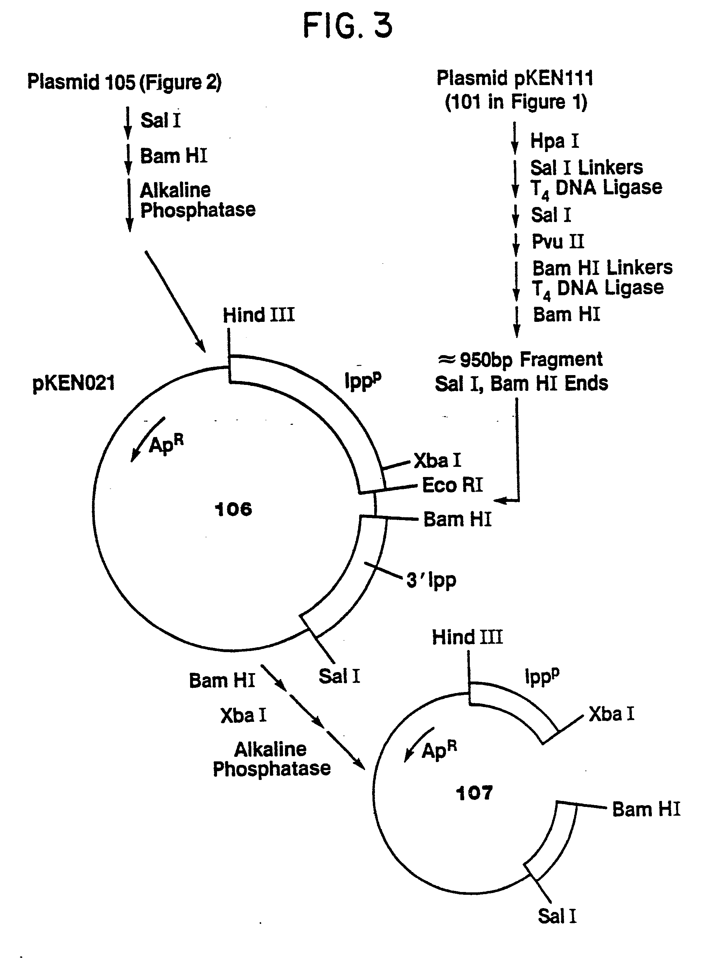

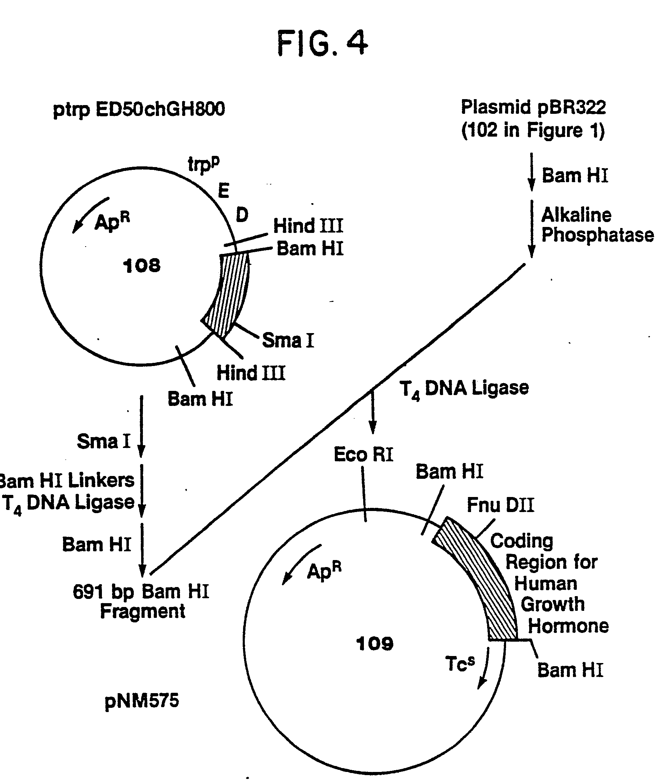

The plasmid pCZ101 starting material is ~10.8 kb and is constructed by ligating the ~0.6 kb XbaI-BamHI fragment of plasmid pNM789B into similarly digested plasmid pIM-I'-A3. The latter plasmid, which contains the transcriptional and translational activating sequence of the E. coli lipoprotein gene and a thermoinducible runaway replicon, can be obtained from E. coli K12 RV308/pIM-I'-A3, a strain deposited and made part of the permanent stock culture collection of the Northern Regional Research Laboratory, Peoria, Illinois. The strain is available as a preferred source and stock reservoir of the plasmid under the accession number NRRL B-15733. The plasmid pNM789B starting material is derived from plasmid pKEN111 in accordance with the steps illustrated and described in Figures 1-6 and Example 1 below. Plasmid pKEN111 can be obtained from E. coli K12 CC620/pKEN111, a strain deposited and made part of the permanent stock culture collection of the Northern Regional Research Laboratory, Peoria, Illinois. The strain is available as a preferred source and stock reservoir of the plasmid under the accession number NRRL B-15011. Plasmid pNM789B also contains the transcriptional and translational activating sequence of the E. coli lipoprotein gene and, in addition, the coding sequence, including an appropriately positioned translational stop signal, for a fusion protein comprising bGH and a nine member polypeptide at the bGH N-terminus. Ligation of the aforementioned transcriptional activating and fusion protein-coding sequence, contained in the XbaI-BamHI fragment, to appropriately cleaved plasmid pIM-I'-A3 results in the aforementioned plasmid pCZ101 starting material.

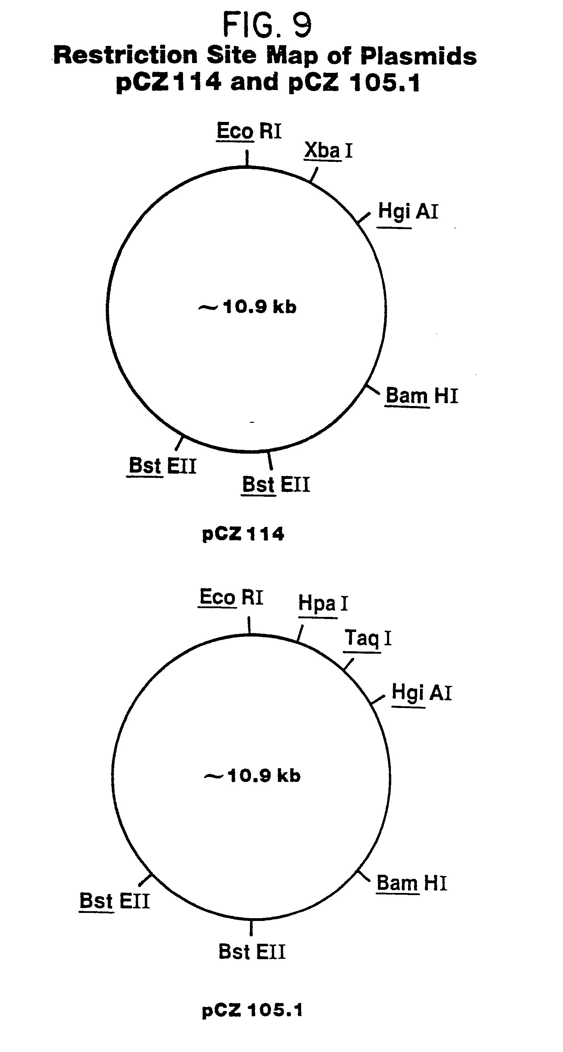

Those skilled in the art will recognize that the XbaI-EgiAI DNA linker described above is an important component of illustrative plasmid pCZ114. This linker encodes 1) a translational activating sequence which is in the reading frame of a nucleotide sequence that simultaneously codes for a peptide and a ribosome binding site 2) a translational stop signal appropriately positioned and in the reading frame of the aforementioned peptide coding sequence; and 3) a translational start signal which is immediately adjacent and downstream from the aforementioned stop signal and which is in the reading frame of a portion of the coding sequence for met-bGH. The remainder of the met-bGH coding sequence (including an appropriately positioned translational stop signal) and a transcriptional activating sequence can be readily provided by ligation, as described above, of the appropriate fragment of plasmid pCZ101. Such a ligation results in the illustrative ~10.8 kb methionyl-bovine growth hormone (met-bGH) expression plasmid pCZ114. Skilled artisans will recognize that the first peptide and the met-bGH functional polypeptide do not comprise a fused gene product,
but rather, are translated as separate proteins from a
single polycistronic mRNA transcript.  A restriction
site map of illustrative plasmid pCZ114 is presented in
Figure 9 of the accompanying drawings.

        Many related bGH expression plasmids which
further exemplify the present invention can also be
constructed.  For example, although a particular DNA
linker sequence must allow for the expression of at
least two separate polypeptides from a single mRNA
transcript, the number of linker DNA sequences that can
be used for such constructions is virtually limitless.
These sequences can be conventionally synthesized by the
modified phosphotriester method using fully protected
dideoxyribonucleotide building blocks.  Such synthetic
methods are well known in the art and can be carried out
in substantial accordance with the procedure of Itakura
et al., 1977, Science 198:1056 and Crea et al., 1978,
Proc. Nat. Acad. Sci. USA 75:5765.  Illustrative
sequences include, but are not limited to, the deoxyribonucleotides identified below as follows:

```
1.  5' CTAGAGGGTATTAATA ATG TTC CCA TTG GAT GAT GAT TAA
       !!!!!!!!!!!!!! !!! !!! !!! !!! !!! !!! !!! !!!
    3'        TCCCATAATTAT TAC AAG GGT AAC CTA CTA CTA ATT

ATG TTC CCA GCC ATG TCC TTG TCC GGC CTG TTT GCC AAC GCT
!!! !!! !!! !!! !!! !!! !!! !!! !!! !!! !!! !!! !!! !!!
TAC AAG GGT CGG TAC AGG AAC AGG CCG GAC AAA CGG TTG CGA

GTGCT 3'
    !
    C      5'
```

```
2. 5' CTAGAGGGTATTAATA ATG GAT CAG GAG GAT TAA ATG TTT
      !!!!!!!!!!!!!! !!! !!! !!! !!! !!! !!! !!! !!!
   3'        TCCCATAATTAT TAC CTA GTC CTC CTA ATT TAC AAA

CCT GCT ATG TCC TTG TCC GGC CTG TTT GCC AAC GCT GTGCT 3'
!!! !!! !!! !!! !!! !!! !!! !!! !!! !!! !!! !!! !
GGA CGA TAC AGG AAC AGG CCG GAC AAA CGG TTG CGA C      5'
```

```
3. 5' CTAGAGGGTATTAATA ATG GAT CAG TAA ATG TTT CCG GCT
      !!!!!!!!!!!!!! !!! !!! !!! !!! !!! !!! !!! !!!
   3'        TCCCATAATTAT TAC CTA GTC ATT TAC AAA GGC CGA

ATG TCC TTG TCC GGC CTG TTT GCC AAC GCT GTGCT 3'
!!! !!! !!! !!! !!! !!! !!! !!! !!! !!! !
TAC AGG AAC AGG CCG GAC AAA CGG TTG CGA C      5'
```

```
4. 5' CTAGAGGGTATTAATA ATG GAT GAT AAG TAA ATG TTC CCA
      !!!!!!!!!!!!!! !!! !!! !!! !!! !!! !!! !!! !!!
   3'        TCCCATAATTAT TAC CTA CTA TTC ATT TAC AAG GGT

GCC ATG TCC TTG TCC GGC CTG TTT GCC AAC GCT GTGCT 3'
!!! !!! !!! !!! !!! !!! !!! !!! !!! !!! !!! !
CGG TAC AGG AAC AGG CCG GAC AAA CGG TTG CGA C      5'
```

```
5. 5' CTAGAGGGTAAATTCT ATG GAT GAT AAG TAA ATG TTT CCG
      !!!!!!!!!!!!!! !!! !!! !!! !!! !!! !!! !!! !!!
   3'        TCCCATTTAAGA TAC CTA CTA TTC ATT TAC AAA GGC

GCT ATG TCC TTG TCC GGC CTG TTT GCC AAC GCT GTGCT 3'
!!! !!! !!! !!! !!! !!! !!! !!! !!! !!! !!! !
CGA TAC AGG AAC AGG CCG GAC AAA CGG TTG CGA C      5'
```

The above designated sequences can be independently ligated to the ~10.2 kb BamHI-XbaI and ~0.6 kb BamHI-HgiAI fragments of plasmid pCZ101 to respectively construct illustrative plasmids pCZ101.1, pCZ1000, pCZ1000.1, pCZ1060 and pCZ1120. These plasmids, upon transcription, provide for a single polycistronic mRNA that codes for both a first peptide and also met-bGH.

The present invention is in no way limited to the use of a particular transcriptional activating sequence since the choice of a specific sequence is not critical to the operability of the present invention. Transcriptional activating sequences which can be substituted for the previously exemplified lipoprotein activating sequence include, but are not limited to, the E. coli tryptophan (trp), E. coli lactose (lac), bacteriophage λ $P_LO_L$, bacteriophage λ $P_RO_R$, Bacillus subtilis vegetative (veg) and the Streptomyces azureus thiostrepton resistance (tsr) gene transcriptional activating sequences. In addition, one or more transcriptional activating sequences can be used in tandem, such as, for example, the trp and lac transcriptional activating sequences. All of the aforementioned sequences have been previously characterized and can be constructed either synthetically or from known plasmids.

More particularly, the tryptophan (trp) transcriptional activating sequence can be obtained by EcoRI-ClaI digestion of plasmid pHI7Δ4Δ1. Plasmid pHI7Δ4Δ1 can be constructed in accordance with the teaching of Example 5 below. Because of the multiplicity of TaqI restriction sites in plasmid pHI7Δ4Δ1, the desired EcoRI-TaqI fragment can best be generated

0154539

and amplified by cloning previously ligated pHI7Δ4Δ1 EcoRI-HpaI and HpaI-TaqI fragments into EcoRI-ClaI-digested plasmid pBR322. The resultant ~4.6 kb plasmid, designated as plasmid pNM608, contains the trp transcriptional activating sequence and is thus useful for constructing additional embodiments of the present invention.

Illustrative plasmids comprising the aforementioned trp activating sequence can be constructed by ligating the 1) ~0.29 kb EcoRI-TaqI fragment of plasmid pNM608; 2) ~10 kb EcoRI-BamHI fragment of plasmid pCZ101; 3) ~0.6 kb BamHI-HgiAI fragment of plasmid pCZ101; and 4) any of the deoxyribonucleotide sequences identified below as follows:

```
1. 5' CGACA ATG TTC CCA TTG GAG GAT GAT TAA ATG TTC CCA
        !!! !!! !!! !!! !!! !!! !!! !!! !!! !!! !!!
   3'    TGT TAC AAG GGT AAC CTC CTA CTA ATT TAC AAG GGT

   GCC ATG TCC TTG TCC GGC CTC TTT GCC AAC GCT GTGCT 3'
   !!! !!! !!! !!! !!! !!! !!! !!! !!! !!! !!! !
   CGG TAC AGG AAC AGG CCG GAG AAA CGG TTG CGA C     5'


2. 5' CGACA ATG TTC CCA TTG GAT GAT GAT TAA ATG TTC CCA
        !!! !!! !!! !!! !!! !!! !!! !!! !!! !!! !!! !!!
   3'    TGT TAC AAG GGT AAC CTA CTA CTA ATT TAC AAG GGT

   GCC ATG TCC TTG TCC GGC CTC TTT GCC AAC GCT GTGCT 3'
   !!! !!! !!! !!! !!! !!! !!! !!! !!! !!! !!! !
   CGG TAC AGG AAC AGG CCG GAG AAA CGG TTG CGA C     5'


3. 5' CGACC ATG GAT CAG GAG TAA ATG TTC CCG GCT ATG TCC
        !!! !!! !!! !!! !!! !!! !!! !!! !!! !!! !!! !!!
   3'    TGG TAC CTA GTC CTC ATT TAC AAG GGC CGA TAC AGG

   TTG TCC GGC CTC TTT GCC AAC GCT GTGCT 3'
   !!! !!! !!! !!! !!! !!! !!! !!! !
   AAC AGG CCG GAG AAA CGG TTG CGA C     5'


4. 5' CGACC ATG GAT GAT AAG TAA ATG TTT CCG GCT ATG TCC
        !!! !!! !!! !!! !!! !!! !!! !!! !!! !!! !!! !!!
   3'    TGG TAC CTA CTA TTC ATT TAC AAA GGC CGA TAC AGG

   TTG TCC GGC CTC TTT GCC AAC GCT GTGCT 3'
   !!! !!! !!! !!! !!! !!! !!! !!! !
   AAC AGG CCG GAG AAA CGG TTG CGA C     5'


5. 5' CGACC ATG GAT GAT AAG GAG TAA ATG TTT CCG GCT ATG
        !!! !!! !!! !!! !!! !!! !!! !!! !!! !!! !!! !!!
   3'    TGG TAC CTA CTA TTC CTC ATT TAC AAA GGC CGA TAC

   TCC TTG TCC GGC CTC TTT GCC AAC GCT GTGCT 3'
   !!! !!! !!! !!! !!! !!! !!! !!! !!! !
   AGG AAC AGG CCG GAG AAA CGG TTG CGA C     5'
```

The above-described ligations respectively result in the illustrative ~10.8 kb plasmids pCZ105.1, pCZ105.2, pCZ106.1, pCZ112.1 and pCZ112.2. These plasmids, upon transcription, provide for a single polycistronic mRNA that codes for a first peptide and also met-bGH and thus further exemplify the present invention. A restriction site map of illustrative plasmid pCZ105.1 is presented in Figure 9 of the accompanying drawings.

The present invention is highly versatile such that virtually any nucleotide sequence that codes for a functional polypeptide can be substituted for the met-bGH coding sequence exemplified above. Such coding sequences include, but are not limited to, sequences that code for human growth hormone (hGH), human pre-growth hormone (pre-hGH), porcine growth hormone (pGH), mammalian growth hormone, avian growth hormone, growth hormone releasing-factor, human insulin A chain, human insulin B chain, human proinsulin, human pre-proinsulin, human and non-human interferon, urokinase, tissue plasminogen activator, interleukin II, any polypeptide hormone, any polypeptide enzyme and any bioactive polypeptide of research or commercial value.

More particularly, an illustrative vector exemplifying the expression of hGH can be constructed by ligating both the ~10.2 kb BamHI-XbaI fragment of plasmid pCZ101 and the ~0.5 kb BamHI-FnuDII fragment of plasmid pNM575 into the linker sequence

```
5' CTAG AGGGTATTAATA ATG TTC CCA TTG GAG GAT GAT TAA ATG
        !!!!!!!!!!!! !!! !!! !!! !!! !!! !!! !!! !!! !!!
   3'            TCCCATAATTAT TAC AAG GGT AAC CTC CTA CTA ATT TAC

TTC CCA ACC ATT CCC TTA TCC AGG CTT TTT GAC AAC GCT ATG
!!! !!! !!! !!! !!! !!! !!! !!! !!! !!! !!! !!! !!! !!!
AAG GGT TGG TAA GGG AAT AGG TCC GAA AAA CTG TTG CGA TAC

CTC CG 3'
!!! !!
GAG GC 5',
```

wherein

        A is deoxyadenyl,

        G is deoxyguanyl,

        C is deoxycytosyl and

        T is thymidyl.

The resultant plasmid, designated as plasmid pCZ1140, sequentially contains, 1) the E. coli lipoprotein gene transcriptional and translational activating sequence in the reading frame of a sequence of nucleotides that codes for both a ribosome binding site and a peptide of the sequence MET PHE PRO LEU GLU ASP ASP, wherein

        MET is methionine,

        PHE is phenylalanine,

        PRO is proline,

        LEU is leucine,

        GLU is glutamic acid and

        ASP is aspartic acid;

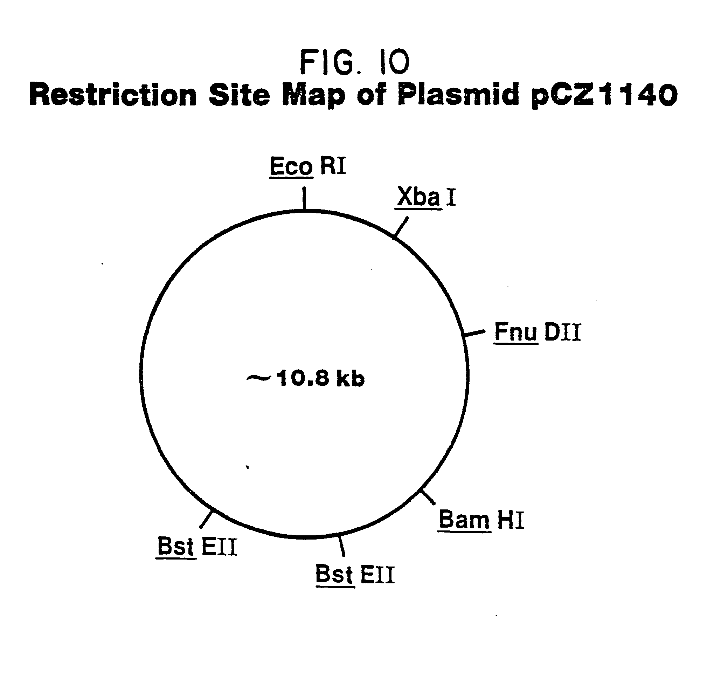

2) a translational stop signal appropriately positioned and in the reading frame of the aforementioned peptide coding sequence; 3) a translational start signal which is immediately adjacent and downstream from the aforementioned stop signal and which is in the reading frame

0154539

of a nucleotide sequence coding for methionyl-human growth hormone (met-hGH); and 4) a translational stop signal appropriately positioned and in the reading frame of the met-bGH coding sequence. The above described linker sequence can be conventionally synthesized in substantial accordance with the previously cited procedure of Itakura et al., 1977 and Crea et al., 1978 and the plasmid pNM575 starting material can be constructed in accordance with the steps illustrated in Figures 1-4 and Example 1 below. A restriction site map of illustrative plasmid pCZ1140 is presented in Figure 10 of the accompanying drawings.

In the specific embodiments herein described, plasmid replication is determined by a thermoinducible runaway replicon disclosed in both GB Patent Publication Number 1,557,774 and Uhlin et al., 1979, Gene 6:91. At temperatures below 30°C, especially 25°C, the replicon maintains a relatively low copy number of about 10-15 copies per cell. When the temperature is raised to 37°C, copy control is lost and plasmid DNA containing the replicon amplifies to 1000-2000 copies per cell. The particular runaway replicon exemplified herein is contained in the previously described plasmid pIM-I'-A3 starting material.

It is understood that the present invention is not limited to the use of any particular runaway replicon or copy number mutant. Other inducible runaway or high copy number replicons can be obtained by appropriate selection or can be constructed in accordance with the procedures disclosed in International Publication Number WO82/02901 and Bittner and Vapnek, 1981, Gene 15:319. In addition, non-runaway replicons can also be used so long as they are functional in E. coli Bacillus, Streptomyces or other suitable microbial host cell. Examples of illustrative replicons include, but are not limited to, replicons from pMB1, ColE1, NR1, RK2, RK6, pSC101, RP1, RP4, F and the like, including bacteriophage that replicate in E. coli; replicons from pEL7, pEL103, SCP2, SCP2* and the like, including bacteriophage that replicate in Streptomyces; and replicons from pC194, pBS1, pE194, pUB110, pT127 and the like, including bacteriophage that replicate in Bacillus. Skilled artisans will appreciate that these and also a variety of runaway replicons can be substituted and used to construct expression vectors that are within the scope of the present invention.

Many other modifications and variations of the present illustrative DNA sequences and plasmids are possible. For example, the degeneracy of the genetic code allows for the substitution of nucleotides in any amino acid coding region as well as for the substitution of the TAG or TGA translational stop signals for the TAA
$$\text{ATC} \quad \text{ACT} \qquad\qquad\qquad\qquad\qquad \text{ATT}$$
translational stop signal specifically exemplified. In addition, the number and choice of nucleotides between the transcriptional activating sequence and the second translational start codon of the vectors can vary as long as appropriate translational signals (ribosome binding site and first translational start and stop signals are provided. Other variations within the present scope include 1) shifting the reading frame of the first translational stop signal relative to the second translational start signal by adding one or more nucleotides; 2) placing one or more nucleotide triplets, thus maintaining the reading frame, between the first translational stop signal and the second translational start signal; and 3) changing the length of the first cistron (region between and including the transcriptional activating sequence and the first translational stop signal) by adding nucleotide triplets between the first translational start and stop signals. Although the length of the cistron is not critical, a relatively short first cistron that codes for 2-20 amino acids is preferred.

Nucleotide triplets comprising the first cistron should be chosen, in accordance with known principles and extrapolations reviewed in Zuker and Stiegler, 1981, Nucleic Acids Research 9(1):133, to avoid generating complementary bases in mRNA. Hydrogen bonding (pairing) between such complementary bases results in stem and loop configurations and folding that are believed to reduce the efficiency of translation. Preferably, the nucleotide triplets comprising the first cistron should also provide, upon transcription, a ribosome binding site appropriately positioned upstream from the second translational start codon. This can be done by selecting nucleotide triplets that generate at least two codons that not only specify amino acids but that also, when taken together, comprise the desired ribosome binding site.

Skilled artisans will understand that all of the modifications and variations disclosed above can be conventionally synthesized in substantial accordance with the synthetic methods previously cited. Therefore, the present invention is in no way limited to the DNA sequences and plasmids specifically exemplified.

The present invention further provides a novel method for producing a functional polypeptide which comprises

1)  transforming a competent cell with a selectable and autonomously replicating recombinant DNA expression vector which comprises

    a)  a transcriptional and translational activating sequence which is in the reading frame of a nucleotide sequence that codes for a peptide or polypeptide, said peptide or polypeptide coding sequence containing a translational stop signal positioned immediately adjacent and downstream from the nucleotide triplet that codes for the carboxy terminus of said peptide or polypeptide,

    b)  a translational start signal which is immediately adjacent and downstream from said stop signal and which is in the reading frame of a nucleotide sequence that codes for a functional polypeptide, said functional polypeptide coding sequence containing a translational stop signal positioned immediately adjacent and downstream from the nucleotide triplet that codes for the carboxy terminus of said functional polypeptide, and

2) culturing the transformed cell under growth conditions suitable for gene expression.

The aforementioned method solves the problem of low level or no gene expression in cases where the mRNA is unstable, lacks an appropriate configuration or lacks the appropriate sequences for the highly efficient attachment of ribosomes. The present invention solves this problem by providing for the formation of a single mRNA transcript comprising two cistrons. The first peptide or polypeptide coding sequence of the vector, which, upon transcription, comprises the first cistron of the message, is designed to avoid the formation of stem and loop secondary structures in the mRNA. Such configurations, which are believed to impede proper ribosomal attachment and thus translation and expression, are avoided by selecting nucleotides that do not generate, upon transcription, complementary bases. Since this can be done in accordance with both the degeneracy of the genetic code and the principles and methods previously cited (Zuker and Stiegler, 1981), the present invention is subject to few, if any, limitations. Therefore, the present method is useful for expressing DNA coding for virtually any polypeptide of research or commercial value. The present invention also can be used to add sequence and thus stabilize an otherwise unstable RNA or to remove or displace sequences that interfere with ribosome binding and the initiation of mRNA translation.

The expression vectors and method of this invention can be used in a wide range of host organisms, for example, gram-negative prokaryotic organisms such as Escherichia coli, for example, E. coli K12, E. coli K12 RV308, E. coli K12 HB101, E. coli K12 C600, E. coli·K12 C600 $R_k$-$M_k$-, E. coli K12 RR1 and E. coli K12 MM294, Serratia, Pseudomonas, and the like; gram-positive prokaryotic organisms such as Bacillus, for example, B. subtilis, B. thuringiensis, and B. thuringiensis var. israelensis, and Streptomyces, for example, S. fradiae, S. ambofaciens, S. lividans and S. griseofuccus. Although all of the embodiments of the present invention are useful, some of the vectors and transformants are preferred. Preferred vectors include pCZ114, pCZ101.1, pCZ105.1, pCZ105.2, pCZ106.1, pCZ112.1, pCZ112.2 and pCZ1140 and preferred transformants include E. coli K12 RV308/pCZ114, E. coli K12 RV308/pCZ101.1, E. coli K12 RV308/pCZ105.1, E. coli K12 RV308/pCZ106.1, E. coli K12 RV308/pCZ112.1, E. coli K12 RV308/pCZ1140. Of this preferred group, plasmids pCZ114 and pCZ101.1 and transformants E. coli K12 RV308/pCZ114 and E. coli K12 RV308/pCZ101.1 are especially preferred.

Those skilled in the art will recognize that the expression vectors and method of this invention are used to transform suitable host organisms such that an exogenous protein product is expressed using standard fermentation conditions. The exogenous protein product is then isolated by routine methods from the resulting fermentation broth. The following examples further illustrate the invention disclosed herein. Both an explanation of and the actual procedures for constructing the invention are described where appropriate.

## Example 1

### Construction of Plasmid pNM789B

A.   Construction of Plasmid pKEN021 and the XbaI-BamHI
     Fragment Thereof

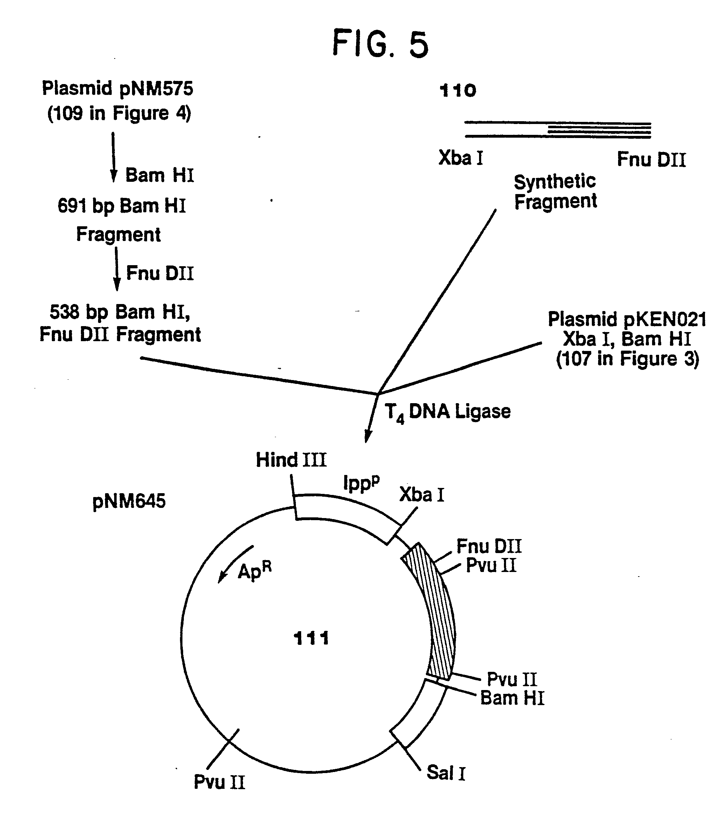

        The ~5.1 kb fragment produced by XbaI-BamHI
cleavage of plasmid pKEN021 (106 in Figure 3) was used
as starting material.  Plasmid pKEN021 is a derivative
of pKEN111, (101 in Figure 1 and further described in
Lee, et al., 1981, J. Bact. 146: 861-866 and Zwiebel,
et al., 1981, J. Bact. 145: 654-656), which is on
deposit in E. coli CC620 (NRRL Deposit No. 15011) and
which has a ~2.8kb fragment which contains the lipoprotein gene of E. coli.  A description of this fragment
is provided in Nakamura and Inouye, 1979, Cell 18:
1109-1117.  In pKEN021, the 650 bp (base pair) sequence
between the unique EcoRI and SalI restriction sites of
pBR322 has been replaced by sequences taken from the
lipoprotein gene of E. coli.  The lipoprotein gene sequence (Nakamura and Inouye, 1979) includes a 462 bp
AluI fragment, upstream from the first triplet (methionine)
of the lipoprotein gene that contains the promoter, the
5' untranslated region and the ribosome binding site.  A
unique XbaI restriction site is located within the
ribosome binding site 16 bp before the translation
initiating methionine signal.  A PvuII restriction site
located 105 bp upstream from the translation termination
codon of the structural gene was changed to a BamHI
restriction site by the addition of a synthetic DNA linker (5'CCGGATCCGG3', obtained from Collaborative Research). The coding sequence for the last thirty-five amino acids of lipoprotein, the translation termination signal, and the sequence corresponding to the 3' untranslated region of the messenger RNA follow the BamHI site. Plasmid pKEN021 also includes some 850 bp of extraneous sequences unrelated to the lipoprotein gene and located downstream of it in the E. coli chromosome. These sequences were included as a consequence of the methods and restriction enzyme sites used in the original isolation of the gene.

Referring to Figures 1, 2, and 3, plasmid pKEN021 is derived from pKEN111 in the following manner: About 50 µg of pKEN111 (101 in Figure 1) are digested with 25 units of HpaII restriction enzyme in 300 µl of a buffer containing 20mM Tris·HCl, pH 7.4, 10mM MgCl$_2$, and 6mM β-mercaptoethanol at 37°C for 2 hours. The mixture is extracted twice with 300 µl of a 50:50 mixture of phenol and chloroform and the recovered aqueous phase is then precipitated with 2.5 volumes of ethanol and 0.1 volumes of 3M sodium acetate. The DNA pellet is dissolved in 100 µl of electrophoresis buffer and fractionated on a 5 percent polyacrylamide gel (acrylamide:bis ratio is 29:1 in all gels except where noted). The gel is stained in a solution containing 0.5 µg/ml of ethidium bromide and bands are visualized under long wave-length ultraviolet light. A 950 bp band is isolated and recovered from the gel by electroelution into a dialysis bag. After phenol/CHCl$_3$ extraction and ethanol precipitation, the recovered DNA (approximately

2.5 µg) is dissolved in 25 µl of TEN (10mM NaCl, 10mM Tris·HCl pH 7.4 and 1mM sodium ethylenedinitrilotetra-acetate (EDTA), pH 8.0).

About 2 µg of the 950 bp HpaII fragment are digested with AluI restriction enzyme in 200 µl of a buffer containing 50mM NaCl, 6mM Tris·HCl (pH 7.6), 6mM $MgCl_2$, and 6mM β-mercaptoethanol for 2 hours at 37°C. The DNA is fractionated on a 6 percent polyacrylamide gel and the 462 bp AluI fragment generated is recovered and purified by the method previously described. The 462 bp AluI fragment (approximately 1 µg) is dissolved in 10 µl of T4 DNA ligase buffer (66mM Tris·HCl pH 7.6, 10mM $MgCl_2$, 10mM dithiothreitol, 0.4mM ATP) containing 150 picomoles of phosphorylated EcoRI linker (5'GGAATTCC3' from Collaborative Research) and 2 units T4 DNA ligase. After incubation at 4°C. for 16 hours, the mixture is heated at 65°C. for 10 minutes and diluted to 100 µl with the addition of EcoRI buffer (100mM Tris·HCl pH 7.2, 50mM NaCl, 10mM $MgCl_2$, 6mM β-mercaptoethanol) and 40 units EcoRI enzyme. After 2 hours at 37°C., the sample is conventionally phenol/$CHCl_3$ extracted and ethanol precipitated. The DNA is then dissolved in 20 µl of T4 DNA ligase buffer containing 0.1 unit T4 DNA ligase and 0.1 µg pBR322 (102 in Figure 1) which has been linearized with EcoRI and then treated with alkaline phosphatase. After ligation at 4°C for 16 hours, the resultant DNA is used to conventionally transform E. coli strain K12 HB101. Transformants are selected on agar plates containing 12 µg/ml of tetracycline and plasmids isolated from resistant colonies by the rapid alkaline extraction procedure described in Birnboim and Doly, 1979, Nucleic Acids Research 7: 1513-1523. A plasmid (103 in Figure 1) containing a 466 bp XbaI-BamHI fragment is selected and used as the starting material for the step next described.

About two μg of this plasmid (103 in Figure 2) are digested with 2 units of HindIII enzyme in 50 μl HindIII buffer (60mM NaCl, 10mM Tris·HCl, pH 7.4, 10mM MgCl$_2$ and 6mM β-mercaptoethanol) for 1 hour at 37°C. After phenol/CHCl$_3$ extraction and ethanol precipitation, the DNA is dissolved in 200 μl of a buffer containing 300mM NaCl, 30mM sodium acetate pH 4.25, 1mM ZnCl$_2$ and 200 units of S1 nuclease (Miles Laboratories). After 1 hour at 15°C., the reaction is stopped by phenol/CHCl$_3$ extraction and ethanol precipitation. The resultant DNA is dissolved in 10 μl T4 DNA ligase buffer containing 20 picomoles phosphorylated BamHI linkers (5'CCGGATCCGG3', from Collaborative Research) and 2 units T4 DNA ligase. After 16 hours at 4°C, the reaction mixture is heated at 65°C for 10 minutes to inactivate the ligase and then diluted to 100 μl in BamHI buffer (150mM NaCl, 20mM Tris·HCl, pH 8.0, 10mM MgCl$_2$, 6mM β-mercaptoethanol) containing 20 units BamHI enzyme. After 2 hours at 37°C, the mixture is purified on a 1 percent agarose gel. The gel is stained and the larger fragment (~4.5 kb) is recovered by elution after freezing and then purified by phenol/CHCl$_3$ extraction and ethanol precipitation. The recovered fragment with BamHI cohesive ends is dissolved in 20 μl of T4 DNA ligase buffer containing 0.1 unit T4 DNA ligase. After 16 hours at 4°C, the DNA is used to transform E. coli HB101. Transformants are selected by resistance to ampicillin ($Ap^r$) at 100 µg/ml and screened for sensitivity to 10 µg/ml tetracycline ($Tc^S$). Several plasmids, prepared by the previously described Birnboim procedure from colonies which are $Ap^rTc^S$, are examined for the absence of a HindIII site and the presence of a single BamHI site. EcoRI, SalI sequential digestion yields a 466 bp and a 305 bp fragment. A plasmid (104 in Figure 2) with these characteristics is selected and then modified to convert the EcoRI site, located upstream of the lpp promoter, to a HindIII restriction site.

Two micrograms of plasmid (104 in Figure 2) are digested in 100 µl of EcoRI buffer with 0.2 units of EcoRI for 10 minutes at 37°C. The reaction is stopped by heating for 10 minutes at 65°C and then, after phenol/$CHCl_3$ extraction, the DNA is ethanol precipitated, dissolved in 200 µl of S1 nuclease buffer containing S1 nuclease at 1000 units/ml and reacted at 12°C for 1 hour. The reaction is stopped by phenol/$CHCl_3$ extraction and ethanol precipitation. The resultant DNA is resuspended in 10 µl of T4 DNA ligase buffer containing 20 picomoles phosphorylated HindIII linker (5'CCAAGCTTGG3', from Collaborative Research) and 2 units of T4 DNA ligase. After 16 hours at 4°C, the mixture is heated for 10 minutes at 65°C, diluted to 150 µl in HindIII buffer containing 10 units HindIII enzyme, incubated for 2 hours at 37°C and then fractionated on a 1 percent agarose gel. The largest band (equivalent to single cut plasmid) is conventionally recovered and purified, dissolved in 20 µl T4 ligase buffer containing 0.2 units T4 ligase, incubated 16 hours at 4°C. and then used to transform E. coli HB101. Transformants are selected for ampicillin resistance and plasmid isolates conventionally analyzed by restriction enzyme analysis. A plasmid (105 in Figure 2) with an EcoRI-HindIII fragment of 500 bp is selected and used as the cloning vector for addition of the 3' region of the lpp gene.

About two µg of plasmid (105 in Figure 3) are digested in 50 µl of SalI restriction buffer (150mM NaCl, 6mM Tris·HCl, pH 7.9, 6mM MgCl$_2$, 6mM β-mercaptoethanol) with 2 units of SalI for 1 hour at 37°C and then diluted to 150 µl in BamHI buffer containing 2 units BamHI. After 1 hour at 37°C, 2.5 units of alkaline phosphatase are added and then incubation is continued for 1 hour at 65°C. The material is phenol/CHCl$_3$ extracted, ethanol precipitated, dissolved in TEN, and used as a cloning vector for the lpp 3' fragment.

To obtain the fragment containing the lpp 3' region, 10 µg of pKEN111 (101 in Figure 3) are digested in 200 µl of HpaI buffer (20mM KCl, 10mM Tris·HCl, pH 7.4, 10mM MgCl$_2$ and 6mM β-mercaptoethanol) with 10 units of HpaI for 2 hours at 37°C. After phenol/CHCl$_3$ extraction and ethanol precipitation, the DNA is dissolved in 10 µl T4 DNA ligase buffer containing 20 picamoles phosphorylated SalI linker (5'GGTCGACC3', from Collaborative Research) and 2 units T4 DNA ligase and then incubated for 16 hours at 4°C. The ligase is inactivated by heating at 65°C for 10 minutes. The resultant material is diluted to 100 µl in SalI buffer containing 10 units of SalI and incubated 1 hour at 37°C, and then diluted to 300 µl in PvuII buffer (60mM NaCl, 6mM Tris·HCl, pH 7.5, 6mM MgCl$_2$, 6mM β-mercaptoethanol) containing 10 units PvuII restriction enzyme. After 1 hour at 37°C, the DNA is fractionated on a 5 percent polyacrylamide gel. Approximately 0.5 µg of a 950 bp fragment is recovered, purified and dissolved in TEN. Two-tenths microgram of the fragment is diluted into 20 µl T4 DNA ligase buffer containing 20 picomoles phosphorylated BamHI linker (5'CCGGATCCGG3', from Collaborative Research) and 2 units T4 DNA ligase and then incubated for 16 hours at 4°C. The resultant DNA is then heated for 10 minutes at 65°C, diluted to 100 µl in BamHI buffer containing 20 units BamHI, incubated at 37°C for 2 hours and then fractionated on a 5 percent polyacrylamide gel to remove excess linker molecules. The resultant 950 bp fragment having BamHI and SalI cohesive ends is conventionally purified and dissolved in 20 µl of T4 DNA ligase buffer containing both 0.2 µg of the cloning vector described previously and 0.2 units T4 DNA ligase. After incubation for 16 hours at 4°C, the DNA is used to transform E. coli K12 HB101. Plasmids are prepared from ampicillin resistant transformants and conventionally analyzed for the SalI-BamHI fragment. The desired plasmid (~5.2 kb) is designated pKEN021 (106 in Figure 3).

Ten micrograms of pKEN021 were digested at 37°C in 200 µl of XbaI/BamHI buffer (150mM NaCl, 10mM Tris·HCl, pH 8, 10mM MgCl$_2$, 6mM β-mercaptoethanol) using

10 units of BamHI for 1 hour followed by 10 units of XbaI for an additional hour at 37°C. The desired XbaI-BamHI-digested DNA was then treated with 2.5 units of alkaline phosphatase for 1.5 hours at 65°C, phenol/CHCl$_3$ extracted, collected by ethanol precipitation, and dissolved in 50 µl of TEN for future use (107 in Figure 3).

B.   Construction of Plasmid pNM575

Plasmid ptrpED50chGH800 (108 in Figure 4), described in Martial et al., 1979, Science 205: 602-607 was used as the source of a DNA fragment containing the coding sequence for a portion of the human growth hormone gene. This fragment can also be constructed synthetically (Itakura et al., 1977 and Crea et al., 1978) or can be obtained using recognized methodology described by Goodman et al., 1979, Methods in Enzymology 68:75-90, by isolating mRNA coding for human growth hormone from human pituitaries. The human growth hormone gene portion of plasmid ptrpED50chGH800 contains a unique SmaI restriction site 6 bp downstream from the translation termination codon of the gene. This site was changed to a BamHI site using the following procedure:  6 µg of the plasmid were digested with 6 units of SmaI in 200 µl of SmaI restriction buffer (15mM Tris·HCl, pH 8.0, 6mM MgCl$_2$, 15mM KCl and 6mM β-mercaptoethanol) for 1.5 hours at 37°C. After digestion was complete, phenol/CHCl$_3$ extraction was performed and the DNA was recovered by ethanol precipitation and then dissolved in 24 µl of TEN.  Forty picomoles of phosphorylated BamHI adapter fragment (Collaborative Research) were added to 0.5 µg (0.2 picomole ends) of the above-digested plasmid in 16 µl of ligase buffer containing 2 units T4 DNA ligase.  The mixture was incubated 2 hours at 22°C, 16 hours at 4°C and then 10 minutes at 65°C.  BamHI cohesive termini were generated by conventional digestion with BamHI restriction enzyme. The enzyme cleaved the linker sequence as well as the BamHI site located at the beginning of the cloned human growth hormone cDNA sequence.  This yielded a 691 bp fragment with cohesive BamHI ends which was separated on a 6 percent polyacrylamide gel and then conventionally recovered.  The recovered DNA fragment was ligated (using 0.2 unit T4 DNA ligase in 20 µl of buffer under previously described conditions) with 0.2 µg of BamHI-digested and alkaline phosphatase-treated pBR322 (102 in Figure 4).  After 16 hours at 4°C, the material was used to transform E. coli strain JA221 (NRRL No. B-15014) in substantial accordance with the transformation procedure of Wensink et al., 1974, Cell 3:315-325. Transformants were selected on agar plates containing 100 µg/ml ampicillin and then plasmids were conventionally isolated and identified by restriction enzyme and gel electrophoretic analysis.  Desired plasmids, designated as pNM575 (109 in Figure 4), contain a BamHI fragment of approximately 700 bp and were conventionally amplified for future use.

0154539

## C. Construction of Plasmid pNM702

The DNA sequence of mature human growth hormone contains one FnuDII site which is 47 bp from the first nucleotide. Twenty-five micrograms of pNM575 were digested in 250 µl of BamHI buffer with 25 units of BamHI at 37°C for 1 hour. The 691 bp fragment with BamHI cohesive termini was conventionally isolated from a 6 percent polyacrylamide gel and purified. After purification of the fragment, one third of it (equivalent to 8 µg of plasmid) was digested in 100 µl of FnuDII buffer (6mM NaCl, 6mM Tris·HCl, pH 7.4, 6mM $MgCl_2$, 6mM β-mercaptoethanol) with 2.5 units FnuDII for 1.5 hours at 37°C. Electrophoresis on a 6 percent polyacrylamide gel and standard recovery procedures were used to isolate a 538 bp DNA fragment containing the coding sequence for the last 175 amino acids of the gene followed by a translation stop signal.

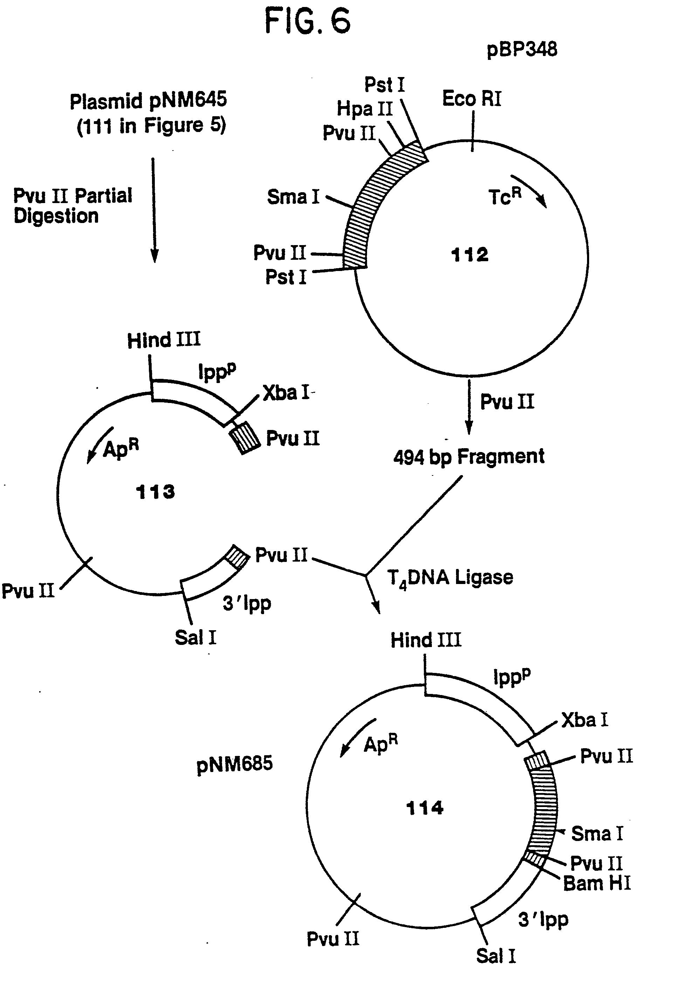

A double stranded DNA fragment (110 in Figure 5) was synthesized by the phosphotriester method to join the lpp promoter region with the human growth hormone coding region. The double stranded DNA fragment (110 in Figure 5) has the following sequence:

XbaI

5'  CTAGAGGGTATTAATAATGTTCCCATTGGATGATGATGATAAGTTCCCAA-
    !!!!!!!!!!!!!!!!!!!!!!!!!!!!!!!!!!!!!!!!!!!!!!!!!!
3'       TCCCATAATTATTACAAGGGTAACCTACTACTACTATTCAAGGGTT-

CCATTCCCTTATCCAGGCTTTTTGACAACGCTATGCTCCG 3'  FnuDII
!!!!!!!!!!!!!!!!!!!!!!!!!!!!!!!!!!!!!!!!!
GGTAAGGGAATAGGTCCGAAAAACTGTTGCGATACGAGGC 5'

The fragment was prepared by recognized phosphotriester methodology by which the following segments were prepared:

1)  CTAGAGGGTAT
2)  TAATAATGTTCC
3)  CATTGGATGAT
4)  GATGATAAGTTCC
5)  CAACCATTCCC
6)  TTATCCAGGC
7)  TTTTTGACAACG
8)  CTATGCTCCG
9)  CATTATTAATACCCT
10) ATGGGAA
11) CTTATCATCATCCA
12) GGTTGGGAA
13) GGATAAGGGAAT
14) GTCAAAAAGCCT
15) CGGAGCATAGCGTT

Using the above-prepared segments, the T4 ligase catalyzed joining reactions were performed stepwise as described below:

0154539

a) 5'-Unphosphorylated segment 1 was joined to 5'-phosphorylated segment 2 in the presence of 5'-phosphorylated segment 9 using T4 ligase to form DNA duplex 1 (Brown et al., 1979, Methods in Enzymology 68:109-151). The duplex was isolated by preparative gel electrophoresis on 15% polyacrylamide.

b) 5'-Phosphorylated segment 3 was joined to 5'-phosphorylated segment 4 in the presence of 5'-phosphorylated segment 11 using T4 ligase to form DNA duplex 2 which was purified by 15% polyacrylamide gel electrophoresis.

c) 5'-Phosphorylated segment 5 was joined to 5'-phosphorylated segment 6 in the presence of 5'-phosphorylated segments 12 and 13 using T4 ligase to form DNA duplex 3 which was purified by 15% polyacrylamide gel electrophoresis.

d) 5'-Phosphorylated segment 7 was joined to 5'-phosphorylated segment 8 in the presence of 5'-phosphorylated segment 14 and 5'-unphosphorylated segment 15 using T4 ligase to form DNA duplex 4 which was purified by 15% polyacrylamide gel electrophoresis.

e) The DNA duplexes 2, 3 and 4 then were joined together by T4 ligase to form DNA duplex 5 which was purified by 15% polyacrylamide gel electrophoresis.

f) 5'-phosphorylated segment 10 and DNA duplex 5 were added, in the presence of T4 ligase, to the DNA duplex 1. The resulting DNA duplex (110 in Figure 5) was purified by 10% polyacrylamide gel electrophoresis. This DNA duplex then was enzymatically phosphorylated using T4 polynucleotide kinase and $[\gamma\text{-}P^{32}]$ATP by following established procedure.

The expression plasmid pNM702 was constructed by enzymatically joining 0.1 picomole (0.4 μg) of the XbaI-BamHI fragment of plasmid pKEN021 (107 in Figure 5), 0.025 picomoles synthetic DNA fragment (110 in Figure 5) and 0.3 picomoles (0.08 μg) of 538 bp fragment (from 109 in Figure 5) in 24 μl of ligation buffer using 1.5 units T4 DNA ligase. After incubation for 16 hours at 4°C, the mixture was used to transform E. coli JA221 as previously described. Transformants were selected on agar plates containing 100 μg/ml ampicillin and were conventionally cultured as a preferred source of the desired expression plasmid.

Expression of human growth hormone was detected by a standard radioimmunoassay procedure (Twomey et al., 1974, Clin. Chem. 20:389-391) and was determined to be at least 2 million molecules per cell.

D.    Construction of Plasmid pNM789

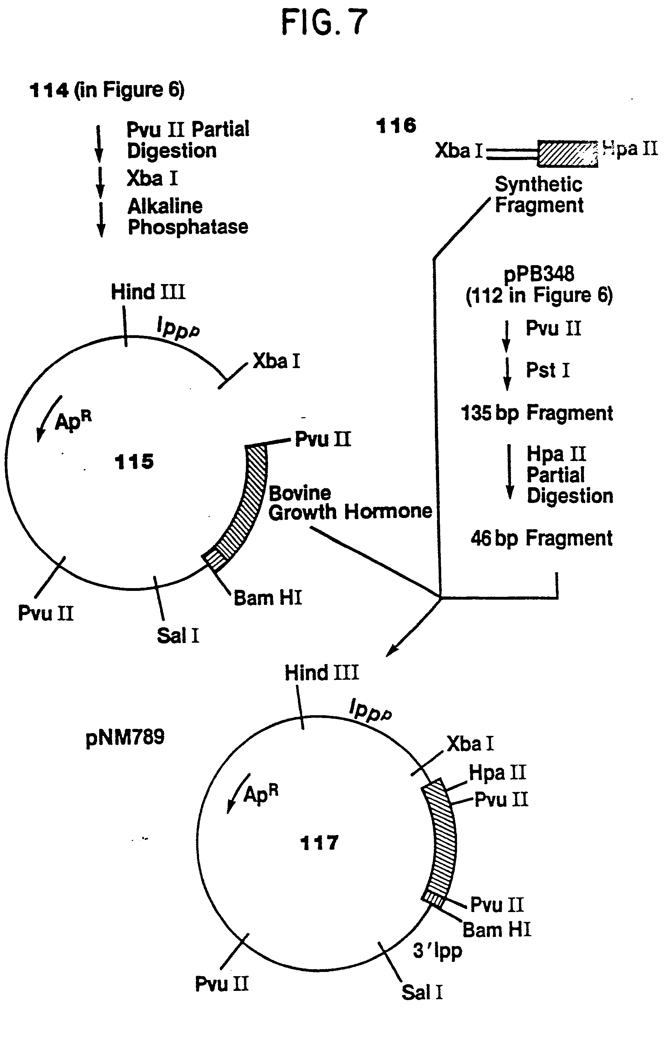

Plasmid pNM702 (111 in Figure 6), the expression plasmid for human growth hormone, was used as the starting material for construction of a plasmid expressing Met-Phe-Pro-Leu-Asp-Asp-Asp-Asp-Lys-bGH.

Plasmid pBP348 (112 in Figure 6), described in Miller et al., 1980, J. Biol. Chem. 255:7521-7524, was used as the source of two DNA fragments containing the coding sequence for a portion of the bovine growth hormone gene. The plasmid contains an 831 bp bovine growth hormone-encoding sequence cloned in the PstI restriction site of pBR322. As an alternative to the method described in Miller et al., 1980, the sequence for bovine growth hormone can be constructed synthetically (Itakura et al., 1977 and Crea et al., 1978) or can also be obtained from messenger RNA isolated from bovine pituitaries by the now routine procedures described by Goodman et al., 1979.

The coding sequences for human growth hormone and bovine growth hormone are very similar and show much homology. Particularly useful in the construction of the expression plasmid for bovine growth hormone were the fragments generated by digestion with the restriction enzyme PvuII. The size of the fragments produced are 497 bp in human growth hormone and 494 bp in bovine growth hormone and the corresponding restriction sites occur in the same reading frames in both sequences.

Ten micrograms of pNM702 (111 in Figure 6) were partially digested with 1 unit of PvuII in 200 μl of PvuII restriction buffer (60mM NaCl, 6mM Tris·HCl, pH 7.5, 6mM MgCl$_2$, 6mM β-mercaptoethanol) for 10 minutes at 37°C. After the reaction was stopped by heating at 65°C for 10 minutes, the DNA was treated with alkaline phosphatase and the fragments separated on a one percent agarose gel. The linear DNA fragment (113 in Figure 6) of the size that corresponded to DNA with the 497 bp PvuII fragment missing (runs slightly faster than single cut plasmid) was conventionally excised, purified and used in the construction of an intermediate plasmid (114 in Figure 6).

A 494 bp PvuII fragment of plasmid pBP348 was prepared by digesting 10 μg of the plasmid in 200 μl

PvuII buffer containing 10 units of PvuII for 1 hour at 37°C. The fragments were separated on a 6 percent polyacrylamide gel and the desired 494 bp fragment (from 112 in Figure 6) was conventionally visualized and purified.

Intermediate plasmid (114 in Figure 6) was constructed by reacting 0.2 μg of the plasmid pNM702 PvuII fragment with 0.05 μg of 494 bp fragment in 20 μl of T4 DNA ligase buffer containing 2 units T4 DNA ligase for 16 hours at 4°C. After transformation and selection of transformants for ampicillin resistance, the plasmids were conventionally analyzed for the presence and proper orientation of the 494 bp PvuII fragment. Plasmids with a 494 bp PvuII fragment and a 440 bp XbaI-SmaI fragment are selected for use in further constructions.

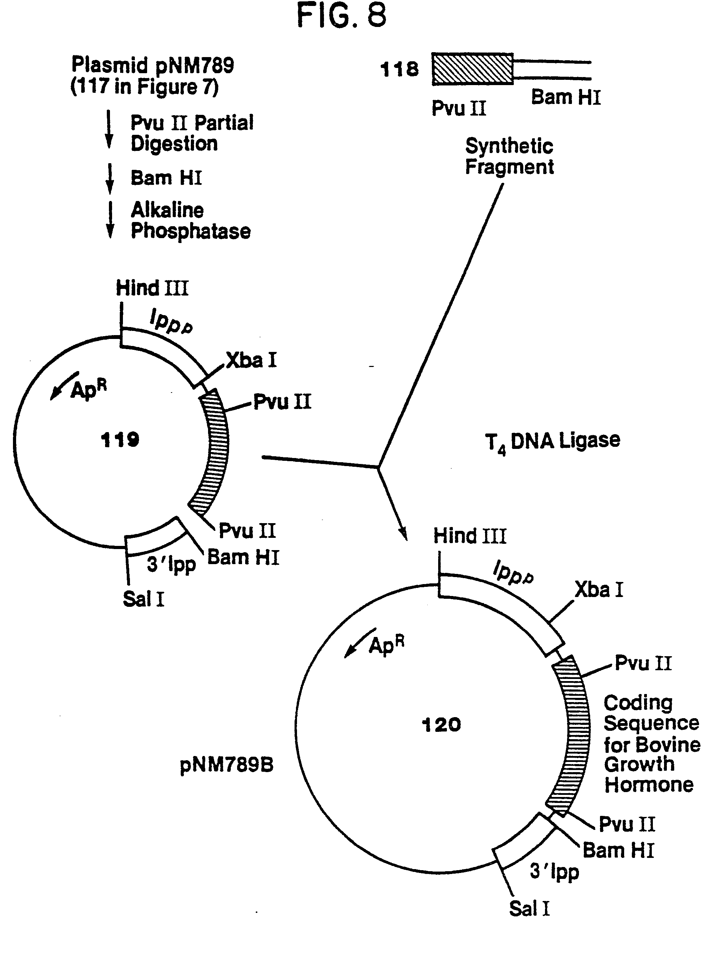

Ten micrograms of the intermediate plasmid (114 in Figure 7) were digested with 1 unit PvuII in 200 μl PvuII buffer for 5 minutes at 37°C. After the heating at 65°C for 10 minutes, the mixture was spread on a 1 percent agarose gel and linear DNA having only a single PvuII cut per molecule was recovered and purified. This recovered material (approximately 3 μg) was digested completely with 5 units of XbaI and treated with alkaline phosphatase. The fragments were spread on a 1 percent agarose gel and the largest fragment (missing the 109 bp fragment between the XbaI and the first PvuII site in human and bovine growth hormone) was conventionally recovered (115 in Figure 7).

The DNA sequence for the first 23 amino acids (69 bp) of bovine growth hormone to the first _PvuII_ site contains 2 _HpaII_ restriction sites, the first of which is 23 bp from the first nucleotide of the coding sequence. A 63 bp fragment (116 in Figure 7) was synthesized by the phosphotriester method. This fragment corresponds to the 19 bp sequence from the _XbaI_ site in the _lpp_ ribosome binding site through the ATG translational start signal followed by the coding sequence for Phe-Pro-Leu-Asp-Asp-Asp-Asp-Lys (24 bp) and 20 nucleotides of the coding sequence of bovine growth hormone (from Phe to the first _HpaII_ site). The fragment has the following sequence:

```
     XbaI

5' CTAGAGGGTATTAATAATGTTCCCATTGGATGATGATGATAAG-
   !!!!!!!!!!!!!!!!!!!!!!!!!!!!!!!!!!!!!!!!!!!!
3'    TCCCATAATTATTACAAGGGTAACCTACTACTACTATTC-

TTCCCAGCCATGTCCTTGTC      3' HpaII
!!!!!!!!!!!!!!!!!!!!
AAGGGTCGGTACAGGAACAGGC    5'
```

In producing the 63 bp fragment, the following nine segments were prepared:

1)    CTAGAGGGTAT

2)    TAATAATGTTCC

3)    CATTGGATGAT

4)    GATGATAAGTTCC

5)    CAGCCATGTCCTTGTC

6)    ATGGGAACATTATTAATACCCT

7)    TTATCATCATCATCCA

8)    ATGGCTGGGAAC

9)    CGGACAAGGAC

Using the above-prepared segments, the T4 ligase catalyzed joining reactions were performed stepwise as described below:

a) 5'-Unphosphorylated segment 1 was joined to 5'-phosphorylated segment 2 in the presence of 5'-phosphorylated segment 6 using T4 ligase to form DNA duplex 1 which was purified by 15% polyacrylamide gel electrophoresis.

b) 5'-Phosphorylated segments 3, 4 and 5 were joined in the presence of 5'-phosphorylated segments 7 and 8 and 5'-unphosphorylated segment 9 using T4 ligase to form DNA duplex 2 which was purified by 15% polyacrylamide gel electrophoresis.

c) Duplexes 1 and 2 then were joined by T4 ligase to form DNA duplex (116 in Figure 7) which was purified by 15% polyacrylamide gel electrophoresis. This DNA duplex then was enzymatically phosphorylated using T4 polynucleotide kinase and $[\gamma-^{P32}]ATP$ following established procedure.

The DNA fragment of 46 bp which runs from the above-described HpaII site to the PvuII site can either be constructed synthetically or obtained from the original pBP348 plasmid. Accordingly, one hundred micrograms of plasmid pBP348 were digested in 400 µl of PvuII buffer with 50 units of PvuII for 2 hours at 37°C. After phenol extraction and ethanol precipitation, the DNA was dissolved in 400 µl of PstI buffer (50mM NaCl, 6mM Tris·HCl, pH 7.4, 6mM $MgCl_2$, 6mM β-mercaptoethanol) with 50 units of PstI for 2 hours at 37°C. The DNA fragments were spread on a 6 percent polyacrylamide gel and the 135 bp fragment containing the desired 46 bp sequence was recovered and purified by standard procedures. One-third of the recovered DNA (equivalent to 33 μg) was subjected to limited digestion by 1 unit of HpaII restriction enzyme in 100 μl HpaII buffer (20mM Tris·HCl, pH 7.4, 7mM MgCl$_2$, 6mM β-mercaptoethanol) for 40 minutes at 37°C. After heating at 65°C for 10 minutes, the DNA fragments were run on a 5 percent acrylamide gel (acrylamide:bis ratio 19:1) along with an appropriate size marker. The desired 46 bp fragment yielded by HpaII partial digestion of the 135 bp fragment (from 112 in Figure 7) was purified by standard procedures.

Two-tenths microgram of the XbaI-PvuII fragment of plasmid vector (115 in Figure 7), 3.2 picomoles of synthetic 63 bp fragment (116 in Figure 7) and 0.5 picomoles 46 bp fragment (from 112 in Figure 7) were incubated in 10 μl ligation buffer with 2 units of T4 DNA ligase for 16 hours at 4°C. The ligation mixture was used to transform E. coli JA221 and the resultant transformants, which thus contained the desired plasmid pNM789, were selected by ampicillin resistance. The identity of plasmid pNM789 (117 in Figure 7) was confirmed by conventionally screening for the presence of both the 494 bp PvuII the 109 bp XbaI-PvuII fragments.

E.    Final Construction of Plasmid pNM789B

Plasmid pNM789 (117 in Figure 8) requires one amino acid codon change for complete conversion to bovine growth hormone.  This was accomplished by the removal of the 28 bp PvuII to BamHI fragment of pNM789 and replacement with a synthetic double stranded fragment having the following sequence (118 in Figure 8):

$$5'\text{CTGTGCCTTCTAG}^{3'}$$
$$\text{!!!!!!!!!!!!!}$$
$$_{3'}\text{GACACGGAAGATCCTAG}_{5'}$$

Ten micrograms of pNM789 were digested with 1 unit of PvuII in 200 µl PvuII buffer for 5 minutes at 37°C.  After heating 10 minutes at 65°C, the mixture was diluted to 300 µl with the addition of BamHI buffer, digested to completion with 10 units of BamHI for 1 hour at 37°C, treated with 5 units of alkaline phosphatase and incubated for 1 hour at 65°C.  The DNA fragments were separated on a 1 percent agarose gel and a DNA fragment (119 in Figure 8) the size of single cut plasmid pNM789 was conventionally purified.  Two-tenths microgram of this fragment was ligated with 5 picomoles of synthetic fragment using 2 units of T4 ligase in 20 µl ligase buffer.  The ligation was carried out overnight at 4°C.  Following transformation, several plasmids were isolated and screened for the appropriate PvuII (494bp) and XbaI-BamHI (628bp) fragments. Plasmids comprising the aforementioned fragments constituted the desired plasmid pNM789B (120 in Figure 8).

## Example 2

Construction of Plasmid pCZ101 and E. coli K12 RV308/-
pCZ101

A.    Isolation of Plasmid pIM-I'-A3

The bacterium E. coli K12/pIM-I'-A3 (NRRL
B-15733) was cultured in TY broth (1% tryptone, 0.5%
yeast extract, 0.5% sodium chloride, pH 7.4) with
50 µg/ml of kanamycin at 25°C according to conventional
microbiological procedures.  After the culture was
diluted 1:10 into fresh broth and after 3 hours incubation at 37°C, about 0.5 ml of the culture was transferred to a 1.5 ml Eppendorf tube and centrifuged for
about 15 seconds.  Unless otherwise indicated, all the
manipulations were done at ambient temperature.  The
resultant supernatant was carefully removed with a
fine-tip aspirator and the cell pellet was resuspended
in about 100 µl of freshly prepared lysozyme solution
(2 µg/ml) which contained 2 µg/ml lysozyme, 50mM
glucose, 10 mM EDTA (diaminetetracetate) and
25 mM Tris·HCl, pH 8.  About 200 µl. of alkaline SDS
(sodium dodecyl sulfate) solution (0.2N NaOH, 1% SDS)
were added and the tube was gently inverted and then
kept at 0°C until lysis was complete (~5 minutes).
Next, about 150 µl of 3M sodium acetate were added and
the contents of the tube mixed gently by inversion for a
few seconds.

The tube was maintained at 0°C for at least 60 minutes and then centrifuged for 15 minutes to yield an almost clear supernatant. The supernatant was transferred to a second centrifuge tube to which 3 volumes of cold 100% ethanol were added. After the tube was held on dry ice ethanol for 5 minutes, the resultant precipitate was collected by centrifugation (5 minutes) and the supernatant was removed by aspiration. The collected pellet was dissolved in 100 μl of TE (10mM Tris·HCl, pH 8.0, 1mM EDTA) and constituted the desired pIM-I'-A3 plasmid DNA.

B.    XbaI-BamHI Digestion of Plasmid pNM789B and
      generation of the ∿0.6 kb XbaI-BamHI Fragment

About 5 μg of plasmid pNM789B DNA in 50 μl Hi Salt buffer* were incubated with 10 units each of BamHI and XbaI restriction enzymes at 37°C for about 1 hour. After the addition of 5 μl of 3M sodium acetate pH 7.0, the DNA was precipitated with 3 volumes of 100% ethanol. The desired DNA digest was dissolved in 100 μl of TE buffer and stored at 0°C for future use.

---

*Hi Salt buffer was conventionally prepared with the following composition:

100 mM NaCl
 20 mM Tris·HCl, pH8.0
 10 mM MgCl$_2$
  5 mM β-mercaptoethanol

C.    XbaI-BamHI Digestion of Plasmid pIM-I'-A3

The desired digestion was carried out in substantial accordance with the procedure of Example 2B except that plasmid pNM789B, rather than plasmid pIM-I'-A3, was used. The desired DNA was dissolved in about 100 µl of TE buffer and stored at 0°C. for future use.

D.    Ligation and Transformation

About 1 µg of the plasmid pIM-I'-A3 digest, 1 µg of the plasmid pNM789B XbaI-BamHI digest, 40 µl water, 5 µl (5mM) ATP, 5 µl ligation mix* and 5 units T4 DNA ligase were incubated at 20°C for about 2 hours. After incubation at 65°C for 2 minutes followed by cooling on ice, the resultant ligation mixture was used to transform, in substantial accordance with the transformation procedure of Wensink, 1974, Cell 3:315, E. coli K12 RV308 on TY plates (1% tryptone, 0.5% yeast extract, 0.5% sodium chloride, 1.5% agar, pH 7.4) containing 50 µg/ml of kanamycin. Bacterial strain E. coli K12 RV308 has been deposited and made part of the permanent stock culture collection of the Northern Regional Research Laboratory, Peoria, Illinois, from which it is available to the public under the accession number NRRL B-15624.

Some of the resultant transformants, as conventionally shown by agarose gel electrophoresis (Maniatis et al., 1982) and other tests, contained only the desired ∿10.8 kb plasmid. Such a transformant, herein designated as E. coli K12 RV308/pCZ101, was selected, plated on TY agar containing appropriate antibiotics and then cultured using conventional micro-biological techniques. The resultant cells were used to isolate plasmid pCZ101 in substantial accordance with the procedure of Example 1A.

---

*Ligation mix can be prepared with the following composition:

500 mM Tris·HCl, pH 7.8
200 mM Dithiothreitol
100 mM MgCl$_2$

## Example 3

Construction of Plasmid pCZ114 and E. coli K12 RV308/-pCZ114

A.     Construction of the ∿10.2 kb BamHI-XbaI Fragment of Plasmid pCZ101

The desired fragment was constructed in substantial accordance with the teaching of Example 2B except that plasmid pCZ101, rather than plasmid pNM789B, was used. The desired ∿10.2 kb BamHI-XbaI restriction fragments were conventionally separated and isolated by agarose gel electrophoresis (Maniatis et al., 1982) and then dissolved in about 100 μl of TE buffer and stored at 0°C for future use.

B.   Construction of the ~0.6 kb BamHI-HgiAI Fragment of
     Plasmid pCZ101

The desired fragment was constructed in substantial accordance with the teaching of Example 2B except that plasmid pCZ101 and HgiAI restriction enzyme, rather than plasmid pNM789B and XbaI restriction enzyme, were respectively used. The desired ~0.6 kb BamHI-HgiAI restriction fragments were conventionally separated and isolated by agarose gel electrophoresis (Maniatis et al., 1982) and then dissolved in about 100 µl of TE buffer and stored at 0°C for future use.

C.   Construction of the DNA Linker Sequence

```
5' CTAGAGGGTATTAATA ATG TTG GAG GAT GAT TAA ATG TTC CCA GCC
   '''''''''''''''' ''' ''' ''' ''' ''' ''' ''' ''' ''' '''
3'     TCCCATAATTAT TAC AAC CTC CTA CTA ATT TAC AAG GGT CGG

ATG TCC TTG TCC GGC CTG TTT GCC AAC GCT GTGCT  3'
''' ''' ''' ''' ''' ''' ''' ''' ''' ''' '
TAC AGG AAC AGG CCC GAC AAA CGG TTG CGA C      5'
```

The desired linker sequence was conventionally synthesized by the modified phosphotriester method in substantial accordance with the teaching of Itakura et al., 1977 and Crea et al., 1978. The aforementioned synthesis method is also specifically illustrated in Example 1.

D.    Ligation and Transformation

About 20 picomoles of the DNA linker of Example 3C, 1 μg of the plasmid pCZ101 ~10.2 kb BamHI-XbaI fragment and 0.5 μg of the plasmid pCZ101 ~0.6 kb BamHI-EciAI fragment were ligated and the resultant plasmid used to transform E. coli K12 RV308 in substantial accordance with the teaching of Example 2D.

Some of the resultant transformants, as conventionally shown by agarose gel electrophoresis (Maniatis et al., 1982) and other tests, contained only the desired ~10.8 kb plasmid. Such a transformant, herein designated as E. coli K12 RV308/pCZ114, was selected, plated on TY agar containing appropriate antibiotics and then cultured using conventional microbiological techniques. The resultant cells were shown, by SDS gel electrophoresis, RIA and other tests, to express met-bGH at high levels. Because plasmid pCZ114 contains a thermoinducible runaway replicon, maximum expression of met-bGH occurs at culture temperatures of about 37°C.

Example 4

Construction of Plasmid pCZ101.1 and E. coli K12 RV308/-pCZ101.1

The desired constructions are made in substantial accordance with the teaching of Example 3 except that the DNA linker sequence

```
5' CTAGAGGGTATTAATA ATG TTC CCA TTG GAT GAT GAT TAA ATG TTC
   !!!!!!!!!!!!!! !!! !!! !!! !!! !!! !!! !!! !!! !!! !!!
3'      TCCCATAATTAT TAC AAG GGT AAC CTA CTA CTA ATT TAC AAG

   CCA GCC ATG TCC TTG TCC GGC CTG TTT GCC AAC GCT GTGCT  3'
   !!! !!! !!! !!! !!! !!! !!! !!! !!! !!! !!! !!! !!!!!
   GGT CGG TAC AGG AAC AGG CCG GAC AAA CGG TTG CGA C       5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ101.1, are plated on TY agar containing appropriate antibiotics and then conventionally cultured for subsequent production and isolation of plasmid pCZ101.1. The transformants are also shown, by SDS gel electrophoresis, RIA and other tests, to express met-bGH at high levels. Because plasmid pCC101.1 contains a thermoinducible runaway replicon, maximum expression of met-bGH occurs at culture temperatures of about 37°C.

Example 5

Construction of Plasmid pHI7Δ4Δ1

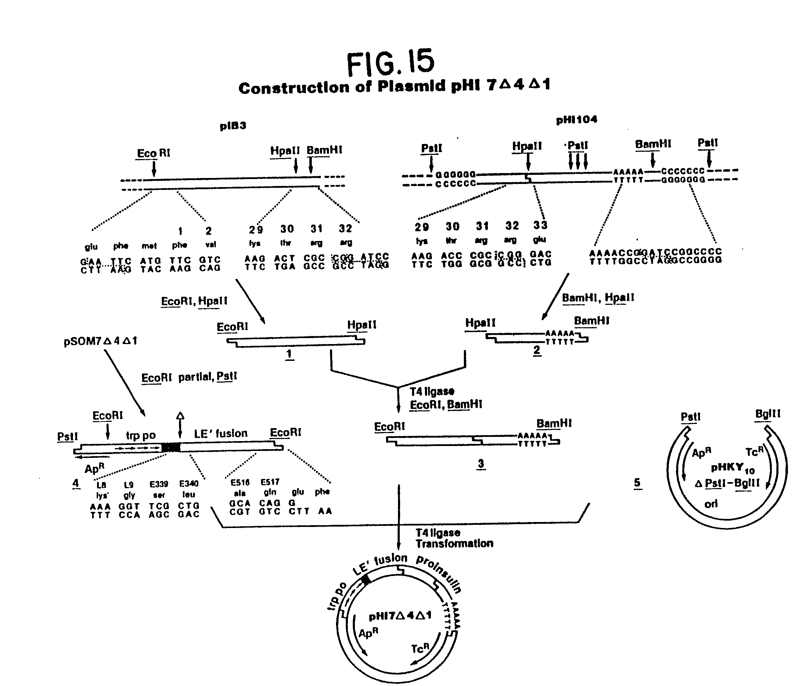

A partial scheme for the construction of plasmid pHI7Δ4Δ1 is presented in Figure 15 of the accompanying drawings.

A.   Construction of Plasmid pBRHtrp

Plasmid pGM1 carries the E. coli tryptophan operon containing the deletion ΔLE1413 (Miozzari, et al., 1978, J. Bacteriology, 1457-1466) and hence expresses a fusion protein comprising the first 6 amino acids of the trp leader and approximately the last third of the trp E polypeptide (hereinafter referred to in conjunction as LE'), as well as the trp D polypeptide in its entirety, all under the control of the trp promoteroperator system.  E. coli K12 W3110tna2trp-Δ102/pGM1 has been deposited with the American Type Culture Collection (ATCC No. 31622) and pGM1 may be conventionally removed from the strain for use in the procedures described below.

About 20 μg of the plasmid were digested with the restriction enzyme PvuII which cleaves the plasmid at five sites.  The gene fragments were next combined with EcoRI linkers (consisting of a self complementary oligonuclectide of the sequence:  pCATGAATTCATG) providing an EcoRI cleavage site for later cloning into a plasmid containing an EcoRI site.  The 20 μg of DNA fragments obtained from pGM1 were treated with 10 units T4 DNA ligase in the presence of 200 pico moles of the 5'-phosphorylated synthetic oligonucleotide pCATGAATTCATG and in 20 μl T4 DNA ligase buffer (20 mM Tris, pH 7.6, 0.5 mM ATP, 10 mM MgCl$_2$, 5 mM dithiothreitol) at 4°C overnight.  The solution was then heated 10 minutes at 70°C to halt ligation.  The linkers were cleaved by EcoRI digestion and the fragments, now with EcoRI ends, were separated using 5 percent polyacrylamide gel electrophoresis (herein after "PAGE"). The three largest fragments were isolated from the gel by first staining with ethidium bromide and then locating the fragments with ultraviolet light and cutting from the gel the portions of interest. Each gel fragment, with 300 μl 0.1xTBE, was placed in a dialysis bag and subjected to electrophoresis at 100 v for one hour in 0.1xTBE buffer (TBE buffer contains: 10.8 gm Tris base, 5.5 gm boric acid, .09 gm $Na_2EDTA$ in 1 liter $H_2O$). The aqueous solution was collected from the dialysis bag, phenol extracted, chloroform extracted, and made 0.2M with respect to sodium chloride. The DNA was then recovered in water after ethanol precipitation. The trp promoter/operator-containing fragments with EcoRI sticky ends were identified by insertion into a tetracycline sensitive plasmid which, upon promoter/operator insertion, becomes tetracycline resistant. All DNA fragment isolations hereinafter described in this example are performed using PAGE followed by the electroelution method described above.

B.    Construction of Plasmid pBRH trp Expressing Tetracycline Resistance Under the Control of the Trp Promoter/Operator and Identification and Amplification of the Trp Promoter/Operator-Containing DNA Fragment Isolated in 'A' above.

Plasmid pBRH1, (Rodriguez, et al., 1979, Nucleic Acids Research 6, 3267-3287 and ATCC No. 37070) expresses ampicillin resistance and contains the gene for tetracycline resistance but, there being no associated promoter, does not express that resistance. Cells harboring the plasmid are accordingly tetracycline sensitive. By introducing a promoter/operator system in the EcoRI site, the plasmid will express tetracycline resistance.

Plasmid pBRH1 was digested with EcoRI. The enzyme was removed by phenol extraction followed by chloroform extraction and then the DNA was resuspended in water after ethanol precipitation. The resulting DNA was, in separate reaction mixtures, combined with each of the three DNA fragments obtained in Example 5A above and ligated with T4 DNA ligase as previously described. The DNA present in the reaction mixture was used to transform competent E. coli K12 294 (NRRL B-15625) by standard techniques (Hershfield et al., 1974, Proc. Nat. Acad. Sci. USA 71:3455-3459) and the bacteria were then plated on LB plates (Maniatis et al., 1982) containing 20 µg/ml ampicillin and 5 µg/ml tetracycline.

Several tetracycline-resistant colonies were selected and the plasmid DNA was isolated and designated pBRHtrp. The presence of the desired fragment was confirmed by restriction enzyme analysis. Plasmid pBRH trp expresses β-lactamase, imparting ampicillin resistance, and contains a DNA fragment which includes the trp promoter/operator. The DNA fragment also codes for a first protein, (designated LE'), comprising a fusion of the first six amino acids of the trp leader and approximately the last third of the trp E polypeptide, a

second protein (designated D'), corresponding to approximately the first half of the trp D polypeptide, and a third protein, coded for by the tetracycline resistance gene.

### C.   Construction of Plasmid pSOM7Δ2

Plasmid pBRHtrp was digested with EcoRI restriction enzyme and the resulting fragment, isolated by PAGE and electroelution, was combined with EcoRI-digested plasmid pSOM11 (Itakura et al., 1977, Sci. 198:1056, U.S. Patent No. 4,366,246, G.B. Patent Publication No. 2,007,676A).  The mixture was ligated with T4 DNA ligase and the resulting DNA transformed into E. coli K12 294 as previously described.  Transformant bacteria were selected on ampicillin-containing plates and the resulting ampicillin-resistant colonies were screened by colony hybridization (Gruenstein et al., 1975, Proc. Nat. Acad. Sci. USA 72:3951-3965).  The trp promoter/operator-containing fragment, isolated from pBRHtrp and then radioactively labelled with $P^{32}$, was used as a probe in the above procedure.  Several colonies were shown to be positive by colony hybridization and were therefore selected.  Plasmid DNA was isolated and the orientation of the inserted fragments was determined by restriction analysis using enzymes BglII and BamHI in double digestion.  Colonies containing the desired plasmid with the trp promoter/operator fragment in the proper orientation were grown in LB medium containing 10 μg/ml ampicillin.  The desired plasmid was designated pSOM7Δ2 and was used for subsequent constructions described below.

D.  Construction of Plasmid pTrp24

1.  Construction of a Gene Fragment Coding for the
    Distal Regions of the LE' Polypeptide With
    BglII and EcoRI Restriction Sites Respectively
    at the 5' and 3' Ends of the Coding Strand

Plasmid pSOM7Δ2 was HindIII digested followed by digestion with lambda exonuclease (a 5' to 3' exonuclease) under conditions chosen so as to digest beyond the BglII restriction site within the LE' encoding region. About 20 µg of HindIII-digested pSOM7Δ2 was dissolved in buffer (20mM glycine buffer, pH 9.6, 1mM MgCl$_2$, 1mM β-mercaptoethanol). The resulting mixture was treated with 5 units of lambda exonuclease for 60 minutes at room temperature. The reaction mixture obtained was then phenol extracted, chloroform extracted, and ethanol precipitated.

To create an EcoRI residue at the distal end of the LE' gene fragment, a primer [32]pCCTGTGCATGAT was synthesized by the improved phosphotriester method (Crea et al., 1978) and hybridized to the single stranded end of the LE' gene fragment resulting from lambda exonuclease digestion. The hybridization was performed by dissolving 20 µg of the lambda exonuclease-treated HindIII digestion product of plasmid pSOM7Δ2 in 20 µl H$_2$O and combining with 6 µl of a solution containing approximately 80 picomoles of the 5'-phosphorylated oligonucleotide described above. The synthetic fragment was hybridized to the 3' end of the LE' coding sequence and the remaining single strand portion of the LE' fragment was filled in by Klenow Polymerase I using dATP, dTTP, dGTP and dCTP. Klenow Polymerase I is the fragment obtained by proteolytic cleavage of DNA Polymerase I. It contains the 5' → 3' polymerizing activity, the 3' → 5' exonucleolytic activity, but not the 5' → 3' exonucleolytic activity of the parental enzyme (Kornberg, 1974, W. H. Freeman and Co., SFO, 98).

The reaction mixture was thus heated to 50°C and let cool slowly to 10°C, whereafter 4 μl of Klenow enzyme were added. After 15 minutes incubation at room temperature, followed by 30 minutes incubation at 37°C, the reaction was stopped by the addition of 5 μl of 0.25 molar EDTA. The reaction mixture was phenol extracted, chloroform extracted, and ethanol precipitated. The DNA was subsequently cleaved with the restriction enzyme BglII and the fragments were separated by PAGE. An autoradiogram obtained from the gel revealed a $p32$-labelled fragment of the expected length of approximately 470 bp, which was recovered by electroelution. As outlined, this fragment LE'(d) has a BclII terminus and a blunt end coinciding with the beginning of the primer.

## 2.   Construction of Plasmid pThα1

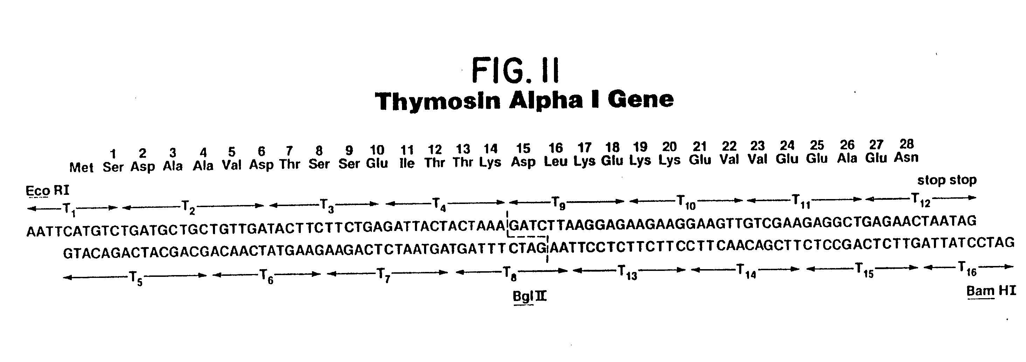

Plasmid pThα1 was constructed by inserting a synthesized gene for thymosin alpha 1 into plasmid pBR322. The synthesis of the thymosin alpha 1 coding DNA involves the synthesis and subsequent ligation of

the 16 oligonucleotides ($T_1$ through $T_{16}$) that are indicated by the double headed arrows in Figure 11 of the accompanying drawings. A met ATG was inserted at the N-terminus and the 5' ends were designed with single-stranded cohesive termini to facilitate joining to plasmids cleaved with EcoR1 and BamH1. As can be readily appreciated, the BglII site in the center of the gene assists in the analysis of recombinant plasmids.

Oligodecxyribonucleotides $T_1$ to $T_{16}$ were synthesized by the modified phosphotriester method of Itakura et al., 1977 and Crea et al., 1978. The various oligodecxyribonuclectides are shown below in Table 1.

0154539

## Table 1

### SYNTHETIC OLIGONUCLEOTIDES FOR THYMOSINα1 GENE

| Compound | Sequence | Length | HPLC Analysis Retention Time (min)* |
|---|---|---|---|
| $T_1$ | A-A-T-T-C-A-T-G-T-C | 10 | 17.4 |
| $T_2$ | T-G-A-T-G-C-T-G-C-T-G-T-T-G-A | 15 | 24.3 |
| $T_3$ | T-A-C-T-T-C-T-T-C-T-G-A | 12 | 20.3 |
| $T_4$ | G-A-T-T-A-C-T-A-C-T-A-A-A | 13 | 22.0 |
| $T_5$ | G-C-A-G-C-A-T-C-A-G-A-C-A-T-G | 15 | 24.8 |
| $T_6$ | G-A-A-G-T-A-T-C-A-A-C-A | 12 | 20.1 |
| $T_7$ | A-G-T-A-A-T-C-T-C-A-G-A-A | 13 | 22.6 |
| $T_8$ | A-A-G-A-T-C-T-T-T-A-G-T | 12 | 20.2 |
| $T_9$ | G-A-T-C-T-T-A-A-G-G-A-G | 12 | 20.4 |
| $T_{10}$ | A-A-G-A-A-G-G-A-A-G-T-T | 12 | 21.1 |
| $T_{11}$ | G-T-C-G-A-A-G-A-G-G-C-T | 12 | 20.5 |
| $T_{12}$ | G-A-G-A-A-C-T-A-A-T-A-G | 12 | 20.4 |
| $T_{13}$ | C-T-T-C-T-T-C-T-C-C-T-T | 12 | 19.9 |
| $T_{14}$ | T-T-C-G-A-C-A-A-C-T-T-C | 12 | 20.5 |
| $T_{15}$ | G-T-T-C-T-C-A-G-C-C-T-C | 12 | 20.2 |
| $T_{16}$ | G-A-T-C-C-T-A-T-T-A | 10 | 17.2 |

*at ambient temperature

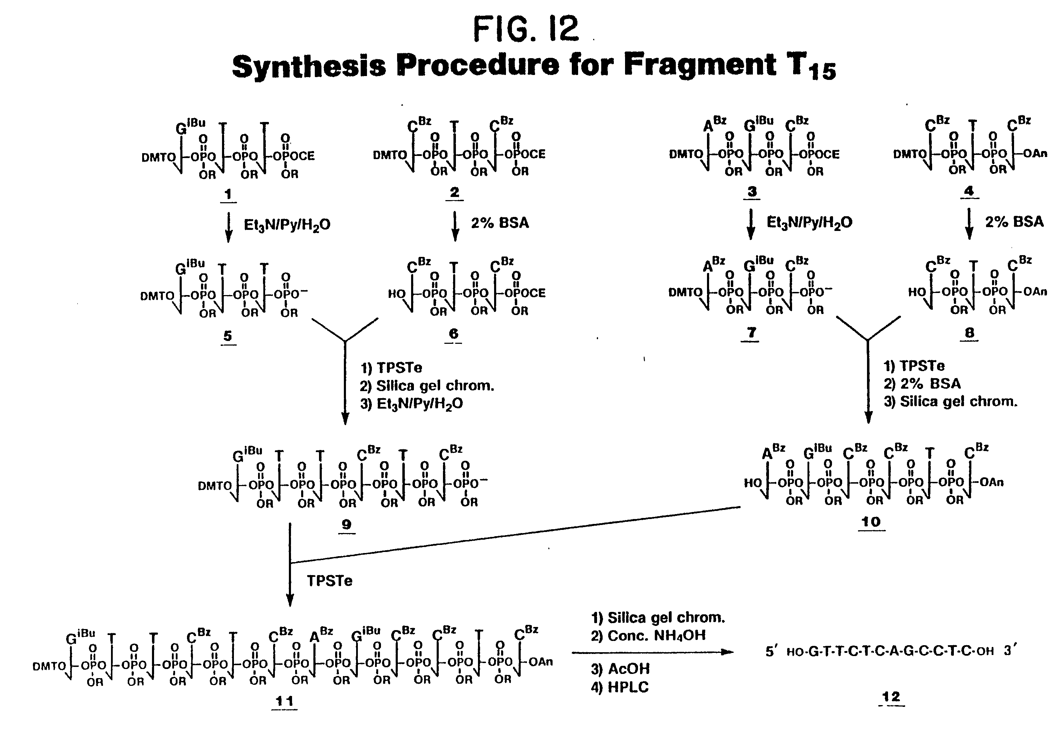

The above synthesis is typified by the following procedure for fragment $T_{15}$ as summarized in Figure 12 of the accompanying drawings. Various nucleotide fragments that are used in the synthesis of T15 are numerically designated in the Figure. The abbreviations employed are as follows: TPSTe, 2,4,6-triisopropylbenzenesulfonyltetrazole; BSA, benzene sulfonic acid; TLC, thin layer chromatography; HPLC, high performance liquid chromatography; DMT, 4,4'-dimethoxytrityl; CE, 2-cyanoethyl; R, p-chlorophenyl; Bz, benzoyl; An, anisoyl; iBu, isobutryl; Py, pyridine; AcOH, acetic acid; $Et_3N$, triethylamine.

The fully protected trideoxyribonucleotides 4 (85 mg, 0.05 mmol) and 2 (180 mg, 0.1 mmol) were deblocked at the 5' hydroxyls by treatment with 2% BSA in 7:3 (v/v) chloroform/methanol (10 and 20 ml, respectively) for 10 minutes at 0°C. Reactions were stopped by addition of saturated aqueous ammonium bicarbonate (2 ml), extracted with chloroform (25 ml) and washed with water (2 x 10 ml). The organic layers were dried (magnesium sulfate), concentrated to small volumes (about 5 ml) and precipitated by addition of petroleum ether (35°-60°C fraction). The colorless precipitates were collected by centrifugation and dried in a dessicator _in vacuo_ to give 6 and 8, respectively, each homogeneous by silica gel tlc (Merck 60 F254, chloroform/methanol, 9:1).

Trimers 1 and 3 (270 mg, 0.15 mmol; 145 mg, 0.075 mmol) were converted into their phosphodiesters (5 and 7) by treatment with triethylamine/pyridine/water (1:3:1, v/v, 10 ml) for 25 minutes at ambient temperature. Reagents were removed by rotary evaporation and the residues dried by repeated evaporations with anhydrous pyridine (3x 10 ml). Trimer 8 (0.05 mmol) and trimer 7 were combined with TPSTe (50 mg, 0.15 mmol) in anhydrous pyridine (3 ml) and the reaction mixture left in vacuo at ambient temperature for two hours. TLC analysis showed that 95% of the trimer 8 had been converted into hexamer product (visualized by detection of the DMT group by spraying with 10% aqueous sulfuric acid and heating at 60°C). The reaction was quenched by addition of water (1 ml) and the solvent evaporated under reduced pressure. After removal of pyridine by coevaporations with toluene, the hexamer was deblocked at the 5' position with 2% BSA (8 ml) as described above for trimers 4 and 2. The product (10) was purified on a silica gel column (Merck 60 H, 3.5 x 5 cm) by step gradient elution with chloroform/methanol (98:2 to 95:5, v/v). Fractions containing product 10 were evaporated to dryness. Similarly, trimer 5 was coupled to 6 and the fully protected product directly purified on silica gel. This latter compound was deblocked at the 3' end by triethylamine/pyridine/water as described above to give fragment 9.

Finally, hexamers 9 and 10 were coupled in anhydrous pyridine (2 ml) with TPSTe (75 mg, 0.225 mmol) as the condensing agent. Upon completion (4 hours, ambient temperature) the mixture was rotary evaporated and the residue chromatographed on silica gel. Product 11 (160 mg) was obtained by precipitation with petroleum ether and appeared homogeneous on TLC.  A portion of compound 11 (20 mg) in pyridine (0.5 ml) was completely deblocked by treatment with concentrated ammonium hydroxide (7 ml, 8 hours, 60°C) and subsequent treatment in 80% acetic acid (15 minutes, ambient temperature).  After evaporation of acetic acid, the solid residue was dissolved in 4% aqueous ammonium hydroxide (v/v, 4 ml) and extracted with ethyl ether (3x2 ml).  The aqueous phase was concentrated to 1-2 ml and a portion applied to HPLC for purification of 12. The fractions corresponding to the major peak were pooled (ca. 2 $A_{254}$ units) and concentrated to about 5 ml.  The final product 12 was desalted on Bio-gel P-2 (1.5 x 100 cm) by elution with 20% aqueous ethanol, reduced to dryness and resuspended in water (200 μl) to give a solution of $A_{254}$ = 10.  The sequence of 12 was confirmed by two-dimensional sequence analysis.

The complete thymosin alpha 1 gene was assembled from the 16 synthetic oligo-nucleotides by methods previously described in detail for somatostatin (Itakura et al., 1977), insulin (Goeddel et al., 1979), and growth hormone (Goeddel et al., 1979, Nature 281:544). Ten microgram quantities of oligonucleotides $T_2$ through $T_{15}$ were quantitatively phosphorylated with [γ-$P^{32}$]-ATP (New England Nuclear) in the presence of T4 polynucleotide kinase (Goeddel et al, 1979), to give specific activities of approximately 1 Ci/mmol.  Radiolabelled fragments were purified by 20% polyacrylamide/7 M urea gel electrophoresis and sequences of the eluted fragments were verified by two-dimensional electrophoresis/

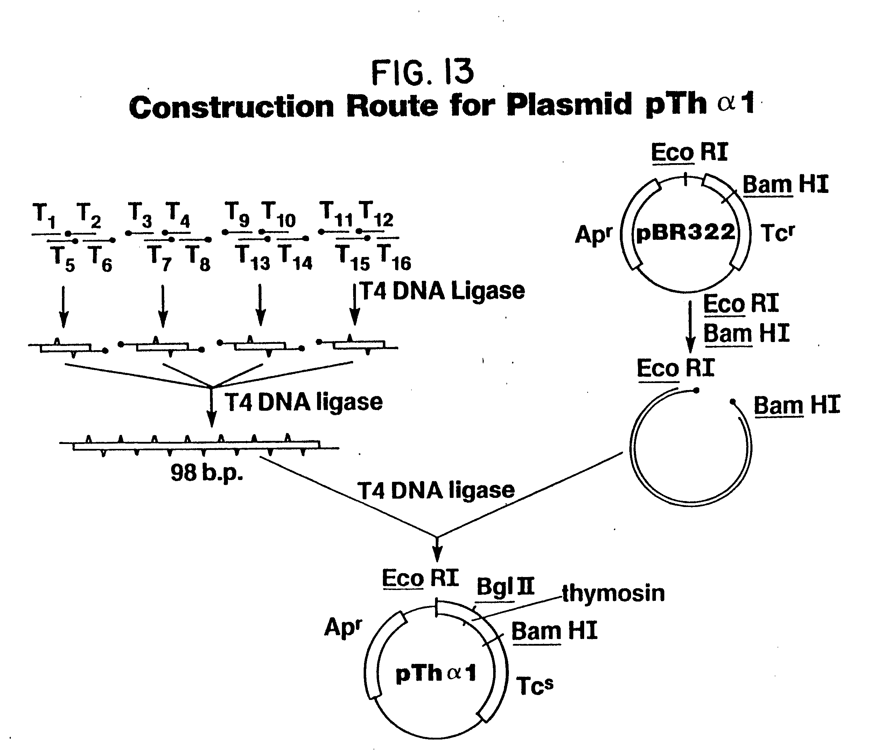

homochromatography (Jay et al., 1974, Nucleic Acids Res. 1:331) of partial snake venom digests. Fragments $T_1$ and $T_{16}$ were left unphosphorylated to minimize undesired polymerization during subsequent ligation reactions. These oligonucleotides (2 μg each) were assembled in four groups of four fragments (see figure 13 of the accompanying drawings), by T4 DNA ligase using published procedures (Goeddel et al., 1979). The reaction products were purified by gel electrophoresis on a 15% polyacrylamide gel containing 7 M urea (Maxam and Gilbert, 1977, Proc. Nat. Acad. Sci. USA 71:3455). The four isolated products were ligated together and the reaction mixture resolved by 10% polyacrylamide gel electrophoresis. DNA in the size range of the thymosin alpha 1 gene (90-105 base pairs) was electroeluted.

Plasmid pBR322 (0.5 μg) was treated with BamHI and EcoRI restriction endonucleases and the fragments separated by polyacrylamide gel electrophoresis. The large fragment was recovered from the gel by electroelution and subsequently ligated to the assembled synthetic DNA (Goeddel et al., Nature 281:544 1979). This mixture was used to transform E. coli K12 294. Five percent of the transformation mixture was plated on LB plates containing 20 μg/ml ampicillin. The four ampicillin resistant colonies obtained were sensitive to tetracycline, suggesting insertion into the tetracycline resistance gene. Analysis of the plasmids from these four colonies showed that in each case the plasmid, designated pThα1, contained (a) a BclII site not found in pBR322 itself, thus indicating the presence of the thymosin alpha 1 gene as shown in Figure 11, and (b) a fragment of approximately 105 base pairs generated by BamHI/EcoRI cleavage. The construction route for plasmid pThα1 (not drawn to scale), is presented in Figure 13 of the accompanying drawings wherein the heavy dots indicate 5'-phosphate groups.

### 3. Reaction of Treated pThα1 and LE'(d) Fragment

The plasmid pThα1 contains a gene specifying ampicillin resistance and a structural gene specifying thymosin alpha 1 cloned at its 5' coding strand end into an EcoRI site and at its 3' end into a BamHI site. The thymosin gene contains a BglII site as well. To create a plasmid capable of accepting the LE'(d) fragment prepared above, pTHα1 was EcoRI digested followed by Klenow polymerase I reaction with dTTP and dATP to blunt the EcoRI residues. BglII digestion of the resulting product created a linear DNA fragment containing the gene for ampicillin resistance and, at its opposite ends, a sticky BglII residue and a blunt end. The resulting product could be recircularized by reaction with the LE'(d) fragment containing a BglII sticky end and a blunt end in the presence of T4 ligase to form the plasmid pTrp24. In doing so, an EcoRI site is recreated at the position where blunt end ligation occurred.

### E.    Construction of Plasmid pSOM7Δ2Δ4

Successive digestion of pTrp24 with BglII and EcoRI, followed by PAGE and electroelution, yields a fragment having codons for the LE'(d) polypeptide with a BglII sticky end and an EcoRI sticky end adjacent to its 3' coding terminus. The LE'(d) fragment can be cloned into the BglII site of plasmid pSOM7Δ2 to form an LE' polypeptide/somatostatin fusion protein expressed under the control of the tryptophan promoter/operator. To do so requires (1) partial EcoRI digestion of pSOM7Δ2 in order to cleave the EcoRI site distal to the tryptophan promoter/operator, and (2) proper choice of the primer sequence in order to properly maintain the codon reading frame, and to recreate an EcoRI cleavage site.

Thus, 16 μg of plasmid pSOM7Δ2 was diluted into 200 μl of buffer containing 20 mM Tris, pH 7.5, 5 mM $MgCl_2$, 0.02% NP40 detergent, and 100 mM NaCl, and treated with 0.5 units EcoRI. After 15 minutes at 37°C, the reaction mixture was phenol extracted, chloroform extracted, ethanol precipitated, and subsequently digested with BglII. The larger resulting fragment was isolated by the PAGE procedure followed by electroelution. This fragment contains the codons "LE'(p)" for the proximal end of the LE' polypeptide, i.e., those upstream from the BglII site. This fragment was next ligated to the above LE'(d) fragment in the presence of T4 DNA ligase to form the plasmid pSOM7Δ2Δ4, which upon transformation into E. coli K12 294, efficiently produced a fusin protein consisting of the fully reconstituted LE polypeptide and somatostatin under the control of the tryptophan promoter/operator.

F.    Construction of Linear DNA Having a PstI Residue at the 3' end and a BglII Residue at its 5' End Bounding a Gene Specifying Tetracycline Resistance

Plasmid pBR322 was HindIII digested and the protruding HindIII ends were digested with S1 nuclease. The S1 nuclease digestion involved treatment of 10 µg of HindIII-cleaved pBR322 in 30 µl S1 buffer (0.3M NaCl, 1 mM $ZnCl_2$, 25 mM sodium acetate, pH 4.5) with 300 units S1 nuclease for 30 minutes at 15°C. The reaction was stopped by the addition of 1 µl of 30X S1 nuclease stop solution (0.8M tris base, 50 mM EDTA). The mixture was phenol extracted, chloroform extracted, ethanol precipitated, and then EcoRI digested as previously described. The resulting fragment, obtained by the PAGE procedure followed by electroelution, has an EcoRI sticky end and a blunt end whose coding strand begins with the nucleotide thymidine. The S1-digested HindIII residue beginning with thymidine can be joined to a Klenow polymerase I-treated BglII residue so as to reconstitute the BglII restriction site upon ligation.

Therefore plasmid pSOM7Δ2, prepared in Example 5C, was BglII digested and the resulting BglII sticky ends were made double stranded by treatment with Klenow polymerase I using all four deoxynucleotide triphosphates. EcoRI cleavage of the resulting product, followed by PAGE and electroelution of the small fragment, yielded a linear piece of DNA containing the tryptophan promoter/operator and codons of the LE' "proximal" sequence upstream from the BglII site ("LE'(p)"). The product had an EcoRI end and a blunt end resulting from filling in the BglII site. However, the BglII site is reconstituted by ligation of the blunt end to the blunt end of the above S1-digested HindIII fragment. Thus, the two fragments were ligated in the presence of T4 DNA ligase to form the recircularized plasmid pHKY10 which was propagated by transformation into competent E. coli K12 294 cells. Tetracycline resistant cells bearing the recombinant plasmid pHKY10 were selected and the plasmid DNA extracted. Digestion with BglII and PstI, followed by isolation by the PAGE procedure and electroelution of the large fragment, yielded the desired linear piece of DNA having PstI and BglII sticky ends. This DNA fragment, thus produced from pHKY10, contains the origin of replication and, therefore, is useful as a component in the construction of plasmid pHI7Δ4Δ1 in which both the genes coding for the trp LE' polypeptide fusion protein and the tetracycline resistance are controlled by the trp promoter/operator.

G.  Construction of Linear DNA Having the Trp Promoter/Operator

Plasmid pSOM7Δ2Δ4, prepared in Example 5E, was subjected to partial EcoRI digestion followed by PstI digestion. The resulting fragment contained the trp promoter/operator and was isolated by the PAGE procedure followed by electroelution. Partial EcoRI digestion was necessary to obtain a fragment which was cleaved adjacent to the 5' end of the somatostatin gene but not
cleaved at the EcoRI site present between the ampicillin
resistance gene and the trp promoter/operator. Ampicillin resistance lost by the PstI cut in the ampicillin
resistance gene can be restored upon ligation with the
final pHKY10 linear DNA derivative produced in
Example 5F above.

H.    Preparation of Synthetic Gene Coding for the
      32 N-Terminal Amino Acids of Proinsulin

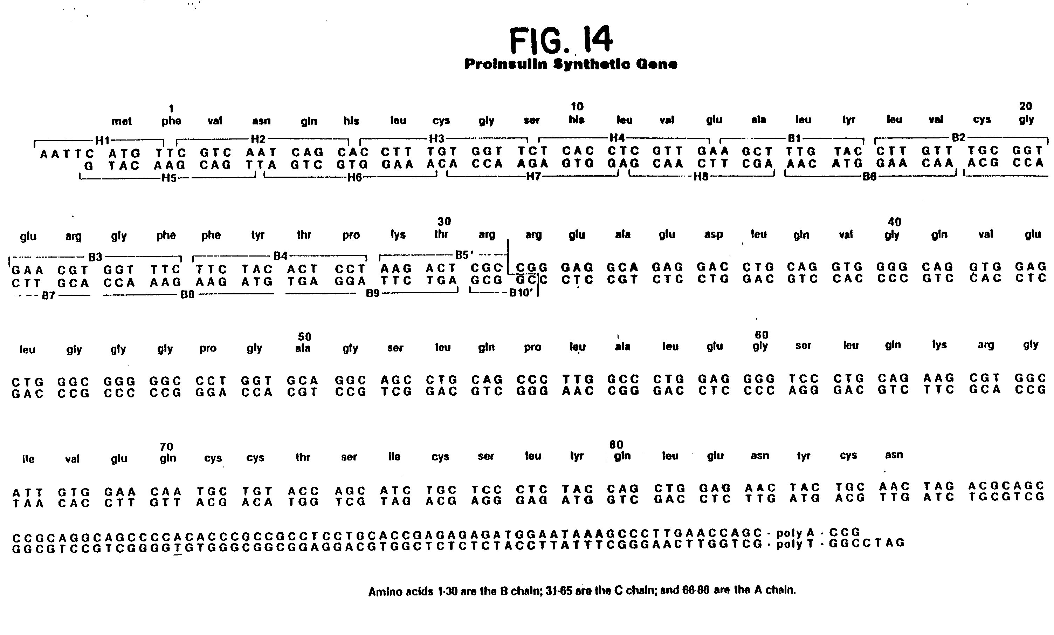

        A series of 18 oligonucleotides, shown in
Table 2, were prepared as a first step in constructing a
gene coding for the first 32 amino acids of proinsulin.
The nucleotide sequence of the entire gene ultimately
constructed is shown in Figure 14.

## Table 2

### Synthetic Oligonucleotides For Human Proinsulin

| Compound | Sequence |
|----------|----------|
| H1 | AATTCATGTT |
| H2 | CGTCAATCAGCA |
| H3 | CCTTTGTGGTTC |
| H4 | TCACCTCGTTGA |
| H5 | TTGACGAACATG |
| H6 | CAAAGGTGCTGA |
| H7 | AGGTGAGAACCA |
| H8 | AGCTTCAACG |
| B1 | AGCTTTGTAC |
| B2 | CTTGTTTGCGGT |
| B3 | GAACGTGGTTTC |
| B4 | TTCTACACTCCT |
| B5' | AAGACTCGCC |
| B6 | AACAAGGTACAA |
| B7 | ACGTTCACCGCA |
| B8 | GTAGAAGAAACC |
| B9 | AGTCTTAGGAGT |
| B10' | GATCCGGCG |

The synthetic nucleotides are shown between brackets and are underlined in Figure 14. These oligonucleotides were synthesized by the conventional method of Crea, et al.., 1978, Itakura, et al., 1975, J. Biol. Chem. 250:4592, and Itakura, et al., 1975, J. Am. Chem. Soc. 97:7327. Some of the oligonucleotides were used for constructing a gene for the human insulin B chain previously described by Crea, et al., 1978 and Goeddel, et al, 1979. Two oligonucleotides (B5' and B10') incorporate HpaII and terminal BamHI and EcoRI restriction sites. The terminal sites are particularly useful for purposes of cloning.

The eight oligonucleotides H1-H8, used previously for constructing the left half of the human insulin B chain gene (Goeddel, et al., 1979), contain the coding sequence for the 1-13 amino acids of the B chain gene and an additional N-terminal methionine. The right half of the B chain gene was constructed from oligonucleotides $B_1$, $B_2$, $B_3$, $B_4$, $B_5'$, $B_6$, $B_7$, $B_8$, $B_9$ and $B_{10}$ by ligation using T4 DNA ligase in substantial accordance with the teaching of Goeddel, et al., 1979. The resultant gene fragment codes for the 14-30 amino acid units of the human insulin B chain and the first arginine of the bridging chain. A HpaII restriction site is incorporated into the gene sequence in the same reading frame and location as the HpaII site in the human insulin gene. After purification of the ligated gene fragment by polyacrylamide gel electrophoresis and after elution of the largest DNA band, the fragment was inserted into HindIII-BamHI-cleaved plasmid pBR322. The resultant plasmid, designated pB3, was inserted into E. coli K12 294 by transformation. The plasmid conferred resistance to antibiotics ampicillin and tetracycline and was found to contain the desired nucleotide sequence.

Two fragments, a 58 base pair HindIII-BamHI fragment of pB3 and a 46 base pair EcoRI-HindIII fragment of pBH1 (disclosed in Goeddel, et al., 1979), were ligated to produce a fragment having EcoRI and BamHI termini. This fragment was conventionally ligated into EcoRI and BamHI restricted plasmid pBR322 and the resultant plasmid, designated pIB3, was then cloned into E. coli K12 294. After conventional amplification and isolation, plasmid pIB3 was digested with EcoRI and HpaII to produce a synthetic gene fragment (Fragment 1, Figure 15) that codes for the N-terminal proinsulin amino acids preceded by a methionine. The synthetic gene was isolated conventionally by polyacrylamide gel electrophoresis.

I.   Isolation of cDNA Coding for the 50 C-Terminal Amino Acids of Human Proinsulin

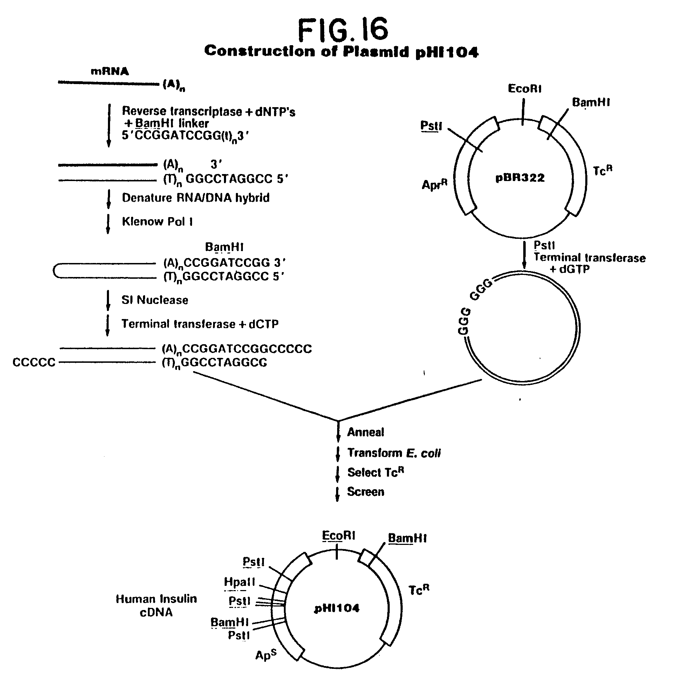

The scheme for obtaining the desired cDNA is presented in Figure 16 of the accompanying drawings. In accordance therewith, the decanucleotide $pCCGGATCCGGTTT_{18}T$, which contained both a BamHI recognition sequence and a 3' polythymidylic acid tract of approximately 20 residues, was synthesized and used to prime AMV reverse transcriptase for cDNA synthesis. The primer was prepared using terminal deoxynucleotidyl transferase (Enzo Biochem, 200 units) with 1 µmol of the BamHI decanucleotide in a reaction volume of 0.6 ml containing $1.5 \times 10^{-4}$ µmol TTP. The reaction was conducted at 37°C for 1 hour in the buffer system of Chang, et al., 1978, Nature 275:617.

Human insulinoma tissue was provided by the Institute für Diabetesforschung, Muenchen, West Germany. Those skilled in the art understand that human insulinoma tissue is readily available and can be obtained from a number of other sources in the medical community. Poly A mRNA (2.5 µg) of the human insulinoma tissue was isolated in substantial accordance with the procedure of Ullrich, et al., 1977, Science 196:1313 and then converted to double stranded cDNA in substantial accordance with the teaching of Wickens, et al., 1978, J. Biol. Chem. 253:2483. Thus, a reaction volume of 80 µl containing 15 mM Tris·HCl (pH 8.3 at 42°C), 21 mM KCl, 8 mM $MgCl_2$, 30 mM B-mercaptoethanol, 2 mM of the primer dCCGGATCCGGTT$_{18}$T, and 1 mM dNTPs was preincubated at 0°C. After addition of AMV reverse transcriptase, the mixture was incubated for 15 minutes at 42°C. The resultant RNA/DNA was then denatured using conventional procedures.

The complementary cDNA strand was synthesized in a reaction volume of 150 µl containing 25 mM Tris·HCl, pH 8.3, 35 mM KCl, 4 mM $MgCl_2$, 15 mM β-mercaptoethanol 1 mM dNTPs and 9 units of Klenow polymerase I. The mixture was incubated at 15°C for 90 minutes followed by 15 hours at 4°C. S1 nuclease digestion was then performed for 2 hours at 37°C using 1000 units of S1 nuclease (Miles Laboratories, Elkhart, Indiana) in substantial accordance with the teaching of Wickens, et al., 1978. The double stranded cDNA (0.37 µg) was electrophoresed on an 8% polyacrylamide gel and DNA fragments larger than 500 base pairs were eluted. Oligodeoxycytidylic acid residues were added to the 3' ends of the fragments using terminal deoxynucleotidyl transferase in substantial accordance with the procedure of Maizel, 1971, Meth. Virol. 5:180. The dC tailed cDNA fragments were then annealed to pBR322 that had first been digested with PstI restriction enzyme and then tailed with deoxyguanidylic acid using terminal deoxynucleotidyl transferase. The resulting plasmids were used to transform E. coli Kl2 294 and the resultant cells plated on LB and TET medium. Colonies resistant to tetracycline but sensitive to ampicillin were isolated and screened for plasmids that had three PstI restriction sites. Such a restriction pattern is indicative of the gene for proinsulin, Sures, et al., 1980, Science 208:57. One plasmid, designated as pHI104, contained a 600 base pair insert, gave the anticipated PstI restriction pattern and contained a BamHI site between the 3' polyA and the polyGC introduced during the cDNA preparation. Some of the nucleotide sequence of the insert is shown in Figure 14.

J.    Assembly of a Gene Coding for Human Proinsulin

The scheme used for assembling a gene coding for human proinsulin is shown in Figure 15 of the accompanying drawings.

The synthetic gene segment coding for the first 31 amino acids of proinsulin, fragment 1 in Figure 15, was recovered from 50 µg of plasmid pIB3 using the restriction endonucleases EcoRI and HpaII as described above. This fragment also contains the ATG sequence for methionine in place of the "presequence" of preproinsulin.

The cDNA gene segment coding for amino acids 32-86, as well as the translation stop signal and the 3' untranslated region of the mRNA, was recovered from 40 µg of plasmid pHI104 by treatment first with BamHI and then HpaII restriction enzymes. The two fragments were isolated by polyacrylamide gel electrophoresis followed by electroelution. The gene fragments were joined by treatment with T4 DNA ligase in 20 µl ligase buffer at 4°C for 24 hours. The mixture was diluted with 50 µl H$_2$O, conventionally extracted with each of phenol and chloroform and then precipitated with ethanol.

The resulting DNA was treated with BamHI and EcoRI restriction enzymes to regenerate these sites and remove gene polymers. The assembled proinsulin gene was isolated by polyacrylamide gel electrophoresis and ligated (using T4 DNA ligase) to EcoRI and BamHI digested plasmid pBR322. The resulting DNA was used to transform E. coli K12 294 and then the resultant colonies were screened for tetracycline sensitivity and ampicillin resistance. Plasmid pHI3, isolated from one such colony, contained the desired proinsulin gene which was subsequently characterized by nucleotide sequence analysis.

K.    Construction of a Plasmid for Expression of a
      Chimeric Protein Containing Human Proinsulin

The complete human proinsulin gene, including the N-terminal codon that codes for methionine, was recovered from plasmid pHI3 by treatment with EcoRI and BamHI restriction enzymes. The desired fragment was purified by gel electrophoresis and then ligated (using T4 DNA ligase) to the plasmid pSOM7Δ2Δ4 PstI-EcoRI partial digest (prepared in Example 5G) and the larger of the PstI-BglII fragments of plasmid pHKY10 (prepared in Example 5F). Thus, about 1 µg of the complete human proinsulin gene with EcoRI and BamHI termini, 4 µg of PstI-EcoRI (partial) pSOM7Δ2Δ4 fragment, and about 1 µg of the PstI-BglII fragment of pHKY10 were ligated at 4°C. for 24 hours using T4 DNA ligase in ligation buffer. The ligated DNA mixture was used to transform E. coli K12 294. Colonies that grew on both ampicillin and tetracycline were selected and were found to contain the desired plasmid pHI7Δ4Δl and to express a protein of the molecular weight expected of the trp LE'- proinsulin fusion. Plasmid pHI7Δ4Δl, which expressed the aforementioned protein, was completely characterized as to the DNA sequence and restriction sites of both the incorporated gene and also the vector. Plasmid pHI7Δ4Δl is depicted in Figure 15 of the accompanying drawings and can be selected readily because of the restoration of ampicillin and tetracycline resistance.

## Example 6

Construction of Plasmid pNM608 and E. coli K12 RV308/pNM608

A.   Construction of the ∿253 bp EcoRI-HpaI Fragment of Plasmid pHI7Δ4Δ1

About 5 µg of plasmid pEI7Δ4Δ1 DNA, 10 µl (10X) reaction buffer*, 80 µl water and 5 µl (5 units) of HpaI restriction enzyme were incubated at 37°C for about 1 hour.  The reaction was terminated by incubation at 70°C for 5 minutes.  After the mixture was cooled on ice, about 12 µl of 1M Tris·HCl, pH 7.2, 7 µl water and 1 µl (10 units) of EcoRI restriction enzyme were added. The resultant mixture was incubated first at 37°C for 1 hour and then at 70°C for 5 minutes.  Following cooling on ice, the resultant DNA was extracted with each of phenol and chloroform:isoamyl alcohol (24:1) and then ethanol precipitated.  The desired ∿253 bp EcoRI-HpaI fragment was conventionally separated by polyacrylamide gel electrophoresis, recovered by electroelution and ETOH precipitation and then dissolved in about 10 µl of TE buffer and stored at 0°C for future use.

\*Reaction buffer (10X) for HpaI restriction enzyme
was prepared with the following composition:

100 mM Tris·HCl, pH 7
100 mM MgCl$_2$
 60 mM β-mercaptoethanol
200 mM KCl

B.    Construction of the ~34 bp HpaI-TaqI Fragment of
      Plasmid pHI7Δ4Δ1

About 20 μg of plasmid pHI7Δ4Δ1 DNA in 32 μl (5X) EcoRI reaction buffer*, 198 μl water and 4 μl EcoRI restriction enzyme (20 units) were incubated at 37°C for about 1 hour. The reaction was terminated by incubation at 70°C for 5 minutes. The resulting 862 bp EcoRI fragment was isolated by electrophoresis on a 6% polyacrylamide gel followed by electroelution and ethanol precipitation. The fragment was then dissolved in about 100 μl of HpaI buffer containing 16 units of HpaI restriction enzyme. After 1.5 hours at 37°C, 7.5 units of TaqI restriction enzyme were added, incubation was continued for an additional 1.5 hours and the resulting fragments conventionally separated on a 7½% polyacrylamide gel. The desired ~34 bp HpaI-TaqI fragment was recovered by electroelution and ethanol precipitation and was then dissolved in 5 μl of TE buffer.

_____

*Reaction buffer (5X) for EcoRI restriction
enzyme was prepared with the following
composition:

250 mM NaCl
500 mM Tris·HCl, pH 7.2
 25 mM MgCl$_2$
 30 mM β-mercaptoethanol

C.    EcoRI-ClaI Digestion of Plasmid pBR322

About 5 μg of plasmid pBR322 DNA, 10 μl of ClaI reaction mix*, 77.5 μl water and 7.5 μl (15 units) of ClaI restriction enzyme were incubated at 37°C for about 1 hour. The reaction was terminated by incubation at 70°C for 5 minutes. After the mixture was cooled on ice, about 12.5 µl of 1M Tris·HCl, pH 7.2, 6.25 µl 1M NaCl, 2.5 µl 0.1M $MgCl_2$ and 1 µl (10 units) of EcoRI restriction enzyme were added. The resultant mixture was incubated first at 37°C for 1 hour and then at 70°C for 5 minutes. Following cooling on ice, the resultant DNA was subjected to electrophoresis on a 1% agarose gel. The large fragment was recovered by elution and ethanol precipitated and then dissolved in about 20 µl of TE buffer and stored at 0°C for future use.

---

*Reaction mix (10X) for ClaI restriction enzyme was prepared with the following composition:

100 mM Tris·HCl, pH 7.4
100 mM $MgCl_2$
 60 mM β-mercaptoethanol

D.    Ligation and Transformation

About 0.2 µg of the ∿253 bp EcoRI-HpaI fragment of Example 6A, 0.5 µg of the HpaI-TaqI fragment of Example 6B and 0.1 µg of EcoRI-ClaI digest of Example 6C were ligated and used to transform E. coli K12 RV308 in substantial accordance with the teaching of Example 2D.

Some of the resultant transformants, as conventionally shown by agarose gel electrophoresis (Maniatis et al, 1982) and other tests, contained only the desired ∿4.6 kb plasmid. Such a transformant, herein designated as E. coli K12 RV308/pNM608, was selected, plated on TY agar containing appropriate antibiotics and then cultured using conventional micro-biological techniques. Plasmid pNM608 comprises and is a preferred source of the ~287 bp EcoRI-ClaI (TaqI) fragment of plasmid pHI7Δ4Δ1.

## Example 7

Construction of Plasmid pCZ105.1 and E. coli K12 RV308/pCZ105.1

A.  EcoRI-ClaI Digestion of Plasmid pNM608 and Generation of the ~0.288 kb EcoRI-TaqI Fragment (Same as the 0.288 kb EcoRI-TaqI Fragment of Plasmid pHI7Δ4Δ1)

About 5 µg of plasmid pNM608 DNA in 50 µl Medium Salt buffer* were incubated with 10 units each of EcoRI and ClaI restriction enzymes at 37°C for about 1 hour. After the addition of 5 µl of 3M sodium acetate, pH 7.0, the DNA was precipitated with 3 volumes of 100% ethanol. The desired DNA digest was dissolved in 100 µl of TE buffer and stored at 0°C for future use.

---

*Medium Salt buffer was prepared with the following composition:

　50 mM NaCl
100 mM Tris·HCl, pH 7.5
　6 mM MgCl$_2$
　2 mM β-mercaptoethanol

0154539

## B.   Construction of the DNA Linker Sequence

```
5' CGACA ATG TTC CCA TTG GAG GAT GAT TAA ATG TTC CCA GCC ATG
    !!! !!! !!! !!! !!! !!! !!! !!! !!! !!! !!! !!! !!! !!!
3'    TGT TAC AAG GGT AAC CTC CTA CTA ATT TAC AAG GGT CGG TAC

TCC TTG TCC GGC CTG TTT GCC AAC GCT GTGCT  3'
!!! !!! !!! !!! !!! !!! !!! !!! !!! !!!!!
AGG AAC AGG CCG GAC AAA CGG TTG CGA C   '''   5'
```

The desired linker sequence is conventionally synthesized by the modified phosphotriester method in substantial accordance with the teaching of Itakura et al, 1977 and Crea et al., 1978. The aforementioned synthesis method is also specifically illustrated in Example 1.


## C.   Ligation and Transformation

About 20 picomoles of the DNA linker of Example 7B, 1 μg of the pNM608 digest of Example 7A, 0.5 μg of the plasmid pCZ101 ∿10.2 kb BamHI-EcoRI fragment (prepared in accordance with the teaching of Example 2B except that EcoRI restriction enzyme and appropriate buffer, rather than XbaI restriction enzyme and buffer, are used), and 1 μg of the plasmid pCZ101 ∿0.6 kb BamHI-HgiAI fragment of Example 3B are ligated and the resultant plasmid used to transform E. coli K12 RV308 in substantial accordance with the teaching of Example 2D.

Some of the resultant transformants, as conventionally shown by agarose gel electrophoresis (Maniatis et al., 1982) and other tests, contain only the desired ∿10.8 kb plasmid. Such a transformant, herein designated as E. coli K12 RV308/pCZ105.1, is

BAD ORIGINAL

selected, plated on TY agar containing appropriate
antibiotics and then cultured using conventional micro-
biological techniques.  The resultant cells are shown,
by SDS gel electrophoresis, RIA and other tests, to
express met-bGH at high levels.  Because plasmid pCZ105.1
contains a thermoinducible runaway replicon, maximum
expression of met-bGH occurs at culture temperatures of
about 37°C.

<u>Example 8</u>

Construction of Plasmid pCZ105.2 and <u>E</u>. <u>coli</u> K12 RV308/-
<u>pCZ105.2</u>

The desired constructions are made in substantial accordance with the teaching of Example 7 except
that the DNA linker sequence

```
5.' CGACA ATG TTC CCA TTG GAT·GAT GAT TAA ATG TTC CCA GCC ATG
     ¦¦¦ ¦¦¦ ¦¦¦ ¦¦¦ ¦¦¦ ¦¦¦ ¦¦¦ ¦¦¦ ¦¦¦ ¦¦¦ ¦¦¦ ¦¦¦ ¦¦¦ ¦¦¦
3'   TGT TAC AAG GGT AAC CTA CTA CTA ATT TAC AAG GGT CGG TAC

TCC TTG TCC GGC CTG TTT GCC AAC GCT GTGCT   3'
¦¦¦ ¦¦¦ ¦¦¦ ¦¦¦ ¦¦¦ ¦¦¦ ¦¦¦ ¦¦¦ ¦¦¦ ¦
AGG AAC AGG CCG GAC AAA CGG TTG CGA C       5'
```

is substituted for the linker sequence of Example 7.
The above specified linker sequence can be constructed
in substantial accordance with the conventional proce-
dure of Itakura <u>et</u> <u>al</u>., 1977 and Crea <u>et</u> <u>al</u>., 1978.  The
aforementioned synthesis method is also specifically
illustrated in Example 1.

The desired transformants, herein designated as E. coli K12 RV308/pCZ105.2, are plated on TY agar containing appropriate antibiotics and then conventionally cultured for subsequent production and isolation of plasmid pCZ105.2. The transformants are also shown, by SDS gel electrophoresis, RIA and other tests, to express met-bGH at high levels. Because plasmid pCZ105.2 contains a thermoinducible runaway replicon, maximum expression of met-bGH occurs at culture temperatures of about 37°C.

## Example 9

### Construction of Plasmid pCZ106.1 and E. coli K12 RV308/- pCZ106.1

The desired constructions are made in substantial accordance with the teaching of Example 7 except that the DNA sequence

```
5' CGACC ATG GAT CAG GAG TAA ATG TTC CCG GCT ATG TCC TTG
         ||| ||| ||| ||| ||| ||| ||| ||| ||| ||| ||| ||| |||
3'    TGG TAC CTA GTC CTC ATT TAC AAG GGC CGA TAC AGG AAC

TCC GCC CTC TTT GCC AAC GCT GTGCT  3'
||| ||| ||| ||| ||| ||| ||| |
AGG CGG GAG AAA CGG TTG CGA C       5'·
```

is substituted for the linker sequence of Example 7. The above specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ106.1, are plated on TY agar containing appropriate antibiotics and then conventionally cultured for subsequent production and isolation of plasmid pCZ106.1. The transformants are also shown, by SDS gel electrophoresis, RIA and other tests, to express met-bGH at high levels. Because plasmid pCZ106.1 contains a thermoinducible runaway replicon, maximum expression of met-bGH occurs at culture temperatures of about 37°C.

### Example 10

### Construction of Plasmid pCZ112.1 and E. coli K12 RV308/-pCZ112.1

The desired constructions are made in substantial accordance with the teaching of Example 7 except that the DNA sequence

```
5' CGACC ATG GAT GAT AAG TAA ATG TTT CCG GCT ATG TCC TTG
      !!! !!! !!! !!! !!! !!! !!! !!! !!! !!! !!! !!! !!!
3'    TGG TAC CTA CTA TTC ATT TAC AAA GGC CGA TAC AGG AAC

TCC GGC CTC TTT GCC AAC GCT GTGCT   3'
!!! !!! !!! !!! !!! !!! !!! !
AGG CCG GAG AAA CGG TTG CGA C        5'
```

is substituted for the linker sequence of Example 7. The above specified linker sequence can be constructed in substantial accordance with the conventional procedure of Itakura et al., 1977 and Crea et al., 1978. The aforementioned synthesis method is also specifically
illustrated in Example 1.

The desired transformants, herein designated
as E. coli K12 RV308/pCZ112.1, are plated on TY agar
containing appropriate antibiotics and then conventionally cultured for subsequent production and isolation of
plasmid pCZ112.1. The transformants are also shown, by
SDS gel electrophoresis, RIA and other tests, to express
met-bGH at high levels. Because plasmid pCZ112.1
contains a thermoinducible runaway replicon, maximum
expression of met-bGH occurs at culture temperatures of
about 37°C.

## Example 11

Construction of Plasmid pCZ112.2 and E. coli K12 RV308/-
pCZ112.2

The desired constructions are made in substantial accordance with the teaching of Example 7 except
that the DNA sequence

```
5' CGACC ATG GAT GAT AAG GAG TAA ATG TTT CCG GCT ATG TCC
        ¦¦¦ ¦¦¦ ¦¦¦ ¦¦¦ ¦¦¦ ¦¦¦ ¦¦¦ ¦¦¦ ¦¦¦ ¦¦¦ ¦¦¦ ¦¦¦
3'    TGG TAC CTA CTA TTC CTC ACC TAC AAA GGC CGA TAC AGG

TTG TCC GGC CTC TTT GCC AAC GCT GTGCT    3'
¦¦¦ ¦¦¦ ¦¦¦ ¦¦¦ ¦¦¦ ¦¦¦ ¦¦¦ ¦¦¦ ¦
AAC AGG CCG GAG AAA CGG TTG CGA C        5'
```

is substituted for the linker sequence of Example 7. The above specified linker sequence can be constructed in accordance with the conventional procedure of Itakura et al., 1977 and Crea et al., 1978. The aforementioned synthesis method is also specifically illustrated in Example 1.

The desired transformants, herein designated as E. coli K12 RV308/pCZ112.2, are plated on TY agar containing appropriate antibiotics and then conventionally cultured for subsequent production and isolation of plasmid pCZ112.2. The transformants are also shown, by SDS gel electrophoresis, RIA and other tests, to express met-bGH at high levels. Because plasmid pCZ112.2 contains a thermoinducible runaway replicon, maximum expression of met-bGH occurs at culture temperatures of about 37°C.

## Example 12

Construction of Plasmid pCZ1000 and E. coli K12 RV308/-pCZ1000

The desired constructions are made in substantial accordance with the teaching of Example 3 except the DNA linker sequence

```
5' CTAGAGGGTATTAATA ATG GAT CAG GAG GAT TAA ATG TTT CCT GCT
   !!!!!!!!!!!!!! !!! !!! !!! !!! !!! !!! !!! !!! !!! !!!
3'     TCCCATAATTAT TAC CTA GTC CTC CTA ATT TAC AAA GGA CGA

ATG TCC TTG TCC GGC CTG TTT GCC AAC GCT GTGCT  3'
!!! !!! !!! !!! !!! !!! !!! !!! !!! !!! !
TAC AGG AAC AGG CCG GAC AAA CGG TTG CGA C      5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ1000, are plated on TY agar containing appropriate antibiotics and then conventionally cultured for subsequent production and isolation of plasmid pCZ1000. The transformants are also shown, by SDS gel electrophoresis, RIA and other tests, to express met-bGH at high levels. Because plasmid pCZ1000 contains a thermoinducible runaway replicon, maximum expression of met-bGH occurs at culture temperatures of about 37°C.

## Example 13

Construction of Plasmid pCZ1000.1 and E. coli K12 RV308/-pCZ1000.1

The desired constructions are made in substantial accordance with the teaching of Example 3 except the DNA linker sequence

```
5'CTAGAGGGTATTAATA ATG GAT CAG TAA ATG TTT CCG GCT ATG TCC
   !!!!!!!!!!!!!!! !!! !!! !!! !!! !!! !!! !!! !!! !!! !!!
3'    TCCCATAATTAT TAC CTA GTC ATT TAC AAA GGC CGA TAC AGG

TTG TCC GGC CTG TTT GCC AAC GCT GTGCT  3'
!!! !!! !!! !!! !!! !!! !!! !!! !
AAC AGG CCG GAC AAA CGG TTG CGA C      5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ1000.1, are plated on TY agar containing appropriate antibiotics and then conventionally cultured for subsequent production and isolation of plasmid pCZ1000.1. The transformants are also shown, by SDS gel electrophoresis, RIA and other tests, to express met-bGH at high levels. Because plasmid pCZ1000.1 contains a thermoinducible runaway replicon, maximum expression of met-bGH occurs at culture temperatures of about 37°C.

## Example 14

Construction of Plasmid pCZ1060 and E. coli K12 RV308/-pCZ1060

The desired constructions are made in substantial accordance with the teaching of Example 3 except the DNA linker sequence

```
5' CTAGAGGGTATTAATA ATG GAT GAT AAG TAA ATG TTC CCA GCC ATG
         !!!!!!!!!!!!! !!! !!! !!! !!! !!! !!! !!! !!! !!! !!!
3'      TCCCATAATTAT TAC CTA CTA TTC ATT TAC AAG GGT CGG TAC

TCC TTG TCC GGC CTG TTT GCC AAC GCT GTGCT   3'
!!! !!! !!! !!! !!! !!! !!! !!! !!!
AGG AAC AGG CCG GAC AAA CGG TTG CGA C         5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ1060, are plated on TY agar containing appropriate antibiotics and then conventionally cultured for subsequent production and isolation of plasmid pCZ1060. The transformants are also shown, by SDS gel electrophoresis, RIA and other tests, to express met-bGH at high levels. Because plasmid pCZ1000 contains a thermoinducible runaway replicon, maximum expression of met-bGH occurs at culture temperatures of about 37°C.

Example 15

Construction of Plasmid pCZ1120 and E. coli K12 RV308/- pCZ1120

The desired constructions are made in substantial accordance with the teaching of Example 3 except the DNA linker sequence

```
5' CTAGAGGGTATTAATA ATG GAT GAT AAG TAA ATG TTT CCG GCT ATG
       !!!!!!!!!!!!!! !!! !!! !!! !!! !!! !!! !!! !!! !!!
3'       TCCCATAATTAT TAC CTA CTA TTC ATT TAC AAA GGC CGA TAC


TCC TTG TCC GGC CTG TTT GCC AAC GCT GTGCT   3'
!!! !!! !!! !!! !!! !!! !!! !!! !!! !
AGG AAC AGG CCG GAC AAA CGG TTG CGA C        5'
```

is substituted for the linker sequence of Example 3C. The above-specified linker sequence can be constructed in substantial accordance with the conventional procedure of Itakura et al., 1977 and Crea et al., 1978. The aforementioned synthesis method is also specifically illustrated in Example 1.

The desired transformants, herein designated as E. coli K12 RV308/pCZ1120 are plated on TY agar containing appropriate antibiotics and then conventionally cultured for subsequent production and isolation of plasmid pCZ1120. The transformants are also shown, by SDS gel electrophoresis, RIA and other tests, to express met-bGH at high levels. Because plasmid pCZ1120 contains a thermoinducible runaway replicon, maximum expression of met-bGH occurs at culture temperatures of about 37°C.

## Example 16

Construction of Plamsid pCZ1140 and E. coli K12 RV308/-pCZ1140

A.    BamHI-FnuDII Digestion of Plasmid pNM575 and Generation of the ~0.5 kb BamHI-FnuDII Fragment

About 5 μg of plasmid pNM575 (prepared in Example 1B) in 50 μl Medium Salt buffer* are incubated with 10 units each of BamHI and FnuDII restriction enzymes at 37°C for about 1 hour. After the addition of 5 μl of 3M sodium acetate, pH 7.0, the DNA is precipitated with 2 volumes of 100% ethanol and recovered. The desired DNA digest is dissolved in 100 µl of TE buffer and stored at 0°C for future use.

---

*Medium Salt buffer is prepared with the following composition:

    50 mM NaCl
    50 mM Tris·HCl, pH 8
    10 mM $MgCl_2$
     6 mM β-mercaptoetanol

B.  Construction of the DNA Linker Sequence

5' CTAGAGGGTATTAATA ATG TTC CCA TTG GAG GAT GAT TAA ATG TTC
   ∶∶∶∶∶∶∶∶∶∶∶∶ ∶∶∶ ∶∶∶ ∶∶∶ ∶∶∶ ∶∶∶ ∶∶∶ ∶∶∶ ∶∶∶ ∶∶∶ ∶∶∶
3'          TCCCATAATTAT TAC AAG GGT AAC CTC CTA CTA ATT TAC AAG

CCA ACC ATT CCC TTA TCC AGG CTT TTT GAC AAC GCT ATG CTC CG  3'
∶∶∶ ∶∶∶ ∶∶∶ ∶∶∶ ∶∶∶ ∶∶∶ ∶∶∶ ∶∶∶ ∶∶∶ ∶∶∶ ∶∶∶ ∶∶∶ ∶∶∶ ∶∶∶ ∶∶
GGT TGG TAA GGG AAT AGG TCC GAA AAA CTG TTG CGA TAC GAG GC  5'

    The above specified linker sequence can be constructed in substantial accordance with the conventional procedure of Itakura et al., 1977 and Crea et al., 1978.  The aforementioned synthesis method is also specifically illustrated in Example 1.

C.  Ligation and Transformation

About 20 picomoles of the DNA linker of Example 16B, 1 µg of the BamHI-FnuDII digest of Example 16A and 0.5 µg of the ∿10.2 kb BamHI-XbaI fragment of Example 3A are ligated and the resultant plasmid used to transform E. coli K12 RV308 in substantial accordance with the teaching of Example 2D.

        Some of the resultant transformants, as
conventionally shown by agarose gel electrophoresis
(Maniatis et al., 1982) contain only the desired
~10.8 kb plasmid.  Such a transformant, herein designated as E. coli K12 RV308/pCZ1140, is selected, plated
on TY agar containing appropriate antibiotics and then
cultured using conventional microbiological techniques.
The resultant cells are shown, by SDS gel electrophoresis, RIA and other tests, to express met-hGH at
high levels.  Because plasmid pCZ1140 contains a
thermoinducible runaway replicon, maximum expression of
met-hGH occurs at culture temperatures of about 37°C.

CLAIMS

1.   A recombinant DNA expression vector which comprises

   a)   a transcriptional and translational activating sequence which is in the reading frame of a nucleotide sequence that codes for a peptide or polypeptide, said peptide or polypeptide coding sequence containing a translational stop signal positioned immediately adjacent and downstream from the nucleotide triplet that codes for the carboxy terminus of said peptide or polypeptide, and

   b)   a translational start signal which is immediately adjacent and downstream from said stop signal and which is in the reading frame of a nucleotide sequence that codes for a functional polypeptide, said functional polypeptide coding sequence containing a translational stop signal positioned immediately adjacent and downstream from the nucleotide triplet that codes for the carboxy terminus of said functional polypeptide,

subject to the limitation that said vector is selectable and autonomously replicating.

2.   The vector of Claim 1 wherein the nucleotide sequence that codes for a peptide or polypeptide contains a functional translational activating sequence.

3.   The vector of Claim 1 or 2 which is a plasmid.

4.   The vector of Claim 1, 2, or 3 in which the peptide or polypeptide coding sequence codes for 2-20 amino acids.

5. The vector of any of Claims 1 to 4 in which the peptide or polypeptide coding sequence codes for MET-PHE-PRO-LEU-GLU-ASP-ASP, MET-GLU-ASP-ASP-LYS, MET-ASP-ASP-LYS, MET-GLU-ASP-GLN or MET-ASP-GLN wherein

MET is methionine,

PHE is phenylalanine,

PRO is proline,

LEU is leucine,

GLU is glutamic acid,

ASP is aspartic acid and

GLN is glutamine.

6. The vector of any of Claims 1 to 5 in which the transcriptional activating sequence is the E. coli tryptophan transcriptional activating sequence, E. coli lipoprotein transcriptional activating sequence, E. coli lactose transcriptional activating sequence, bacteriophage λ $P_LO_L$ transcriptional activating sequence, bacteriophage λ $P_RO_R$, Bacillus subtilis vegetative or Streptomyces azureus thiostrepton resistance transcriptional activating sequence.

7. The vector of Claim 6 in which the transcriptional activating sequence comprises one or more E. coli transcriptional activating sequences in tandem.

8. The vector of Claim 6 or 7 in which the transcriptional activating sequence the E. coli tryptophan transcriptional activating sequence or the E. coli lipoprotein transcriptional activating sequence.

9.  The vector of any of Claims 1 to 8 in which the functional polypeptide coding sequence codes for bovine growth hormone, human growth hormone, human pre-growth hormone, porcine growth hormone, mammalian growth hormone, avian growth hormone, human insulin A chain, human insulin B chain, human proinsulin, human pre-proinsulin, interferon, urokinase, human tissue plasminogen activator, growth hormone releasing factor or interleukin II.

10.  The vector of any of Claims 1 to 9 in which the functional polypeptide coding sequence codes for a bioactive polypeptide.

11.  The vector of any of Claims 1 to 10 which is plasmid pCZ114, pCZ101.1, pCZ1000, pCZ1000.1, pCZ1060, pCZ1120, pCZ105.1, pCZ105.2, pCZ106.1, pCZ112.1, pCZ112.2 or pCZ1140.

12.  The vector of any of Claims 1 to 10 which further comprises one or two nucleotide pairs positioned between the translational stop signal of the peptide or polypeptide coding sequence and the translational start signal of the functional polypeptide coding sequence.

13.  The vector of any of Claims 1 to 10 which further comprises at least one nucleotide triplet positioned between the translational stop signal of the peptide or polypeptide coding sequence and the translational start signal of the functional polypeptide coding sequence.

14.  Plasmid pCZ101.

15.  A cell transformed by the recombinant DNA expression vector of any of Claims 1 to 13.

16.   The cell of Claim 15 which is a prokaryotic cell.

17.   The cell of Claim 16 which is E. coli, Bacillus or Streptomyces.

18.   The cell of Claim 17 which is E. coli K12 RV308/pCZ114, E. coli K12 RV308/pCZ101.1, E. coli K12 RV308/pCZ1000, E. coli K12 RV308/pCZ1000.1, E. coli K12 RV308/pCZ1060, E. coli K12 RV308/pCZ1120, E. coli K12 RV308/pCZ105.1, E. coli K12 RV308/pCZ105.2, E. coli K12 RV308/pCZ106.1, E. coli K12 RV308/pCZ112.1, E. coli K12 RV308/pCZ112.2 or E. coli K12 RV308/pCZ1140.

19.   A method for producing a functional polypeptide which comprises

1)   transforming a cell with a recombinant DNA expression vector of any of Claims 1 to 13 and

2)   culturing the transformed cell under conditions suitable for gene expression.

X-6354-(R)                                    AUSTRIAN

CLAIMS

1. A process for preparing a recombinant DNA expression vector which comprises ligating

a) a transcriptional and translational activating sequence,

b) a DNA linker comprising a nucleotide sequence that codes for a ribosome binding site and for a peptide or polypeptide, said peptide or polypeptide coding sequence containing a translational stop signal positioned immediately adjacent and downstream from the nucleotide triplet that codes for the carboxy terminus of said peptide or polypeptide, and a translational start signal which is immediately adjacent and downstream from said stop signal, and

c) a nucleotide sequence that codes for a functional polypeptide, said functional polypeptide coding sequence containing a translational stop signal positioned immediately adjacent and downstream from the nucleotide triplet that codes for the carboxy terminus of said functional polypeptide,

subject to the limitation that said vector is selectable and autonomously replicating.

2. The process of Claim 1 wherein the vector is a plasmid.

6354-(R)

3. The process of Claim 1 or 2 in which the peptide or polypeptide coding sequence codes for 2-20 amino acids.

4. The process of Claim 1, 2 or 3 in which the peptide or polypeptide coding sequence is a sequence that codes for MET-PHE-PRO-LEU-GLU-ASP-ASP, MET-GLU-ASP-ASP, MET-GLU-ASP-ASP-LYS, MET-ASP-ASP-LYS, MET-GLU-ASP-GLN or MET-ASP-GLN wherein

> MET is methionine,
> PHE is phenylalanine,
> PRO is proline,
> LEU is leucine,
> GLU is glutamic acid,
> ASP is aspartic acid and
> GLN is glutamine.

5. The process of any of Claims 1 to 4 in which the transcriptional activating sequence is the E. coli tryptophan, E. coli lipoprotein, E. coli lactose, bacteriophage λ $P_LO_L$, bacteriophage λ $P_rO_r$, Bacillus subtilis vegetative or Streptomyces azureus thiostrepton resistance transcriptional activating sequence.

6. The process of Claim 5 in which the transcriptional activating sequence comprises one or more E. coli transcriptional activating sequences in tandem.

7. The process of any of Claims 1 to 6 in which the transcriptional activating sequence is the E. coli tryptophan transcriptional activating sequence or the E. coli lipoprotein transcriptional activating sequence.

X-6354-(R)

8.  The process of any of Claims 1 to 7 wherein one or two nucleotide pairs are positioned between the translational stop signal of the peptide or polypeptide coding sequence and the translational start signal of the functional polypeptide coding sequence.

9.  The process of any of Claims 1 to 7 wherein at least one nucleotide triplet is positioned between the translational stop signal of the peptide or polypeptide coding sequence and the translational start signal of the functional polypeptide coding sequence.

10.  The process of any of Claims 1 to 9 wherein the nucleotide sequence that codes for a peptide or polypeptide contains a functional translational activating sequence.

11.  The process of any of Claims 1 to 10 in which the recombinant DNA expression vector comprises a nucleotide sequence that generates, upon transcription, a translatable polycistronic mRNA comprising codons for a functional polypeptide and an unfused additional peptide or polypeptide.

12.  The process of any of Claims 1 to 11 in which the codons for the functional polypeptide and unfused additional peptide or polypeptide are not in the same reading frame.

13.  The process of any of Claims 1 to 12 in which the functional polypeptide coding sequence is a sequence that codes for bovine growth hormone, human growth hormone, human pre-growth hormone, porcine growth hormone, mammalian growth hormone, avian growth hormone,

-6354-(R)

human insulin A chain, human insulin B chain, human proinsulin, human preproinsulin, interferon, urokinase, human tissue plasminogen activator, growth hormone releasing factor or interleukin II.

14. The process of Claim 1, 2 or 3 for preparing plasmid pCZ114 which comprises ligating the ~10.2 kb BamHI-XbaI fragment of plasmid pCZ101, a DNA linker having the nucleotide sequence

```
5' CTAGAGGGTATTAATA ATG TTG GAG GAT GAT TAA ATG TTC CCA
   |||||||||||||||| ||| ||| ||| ||| ||| ||| ||| ||| |||
        TCCCATAATTAT TAC AAC CTC CTA CTA ATT TAC AAG GGT

GCC ATG TCC TTG TCC GGC CTG TTT GCC AAC GCT GTGCT 3'
||| ||| ||| ||| ||| ||| ||| ||| ||| ||| ||| |
CGG TAC AGG AAC AGG CCC GAC AAA CGG TTG CGA C       5'
```

and the ~0.6 kb BamHi-HgiAI fragment of plasmid pCZ101.

15. The process of Claim 1, 2 or 3 for preparing plasmid pCZ101.1 which comprises ligating the ~10.2 kb BamHI-XbaI fragment of plasmid pCZ101, a DNA linker having the nucleotide sequence

```
5' CTAGAGGGTATTAATA ATG TTC CCA TTG GAT GAT GAT TAA ATG TTC
   ||||||||||||||| ||| ||| ||| ||| ||| ||| ||| ||| ||| |||
        TCCCATAATTAT TAC AAG GGT AAC CTA CTA CTA ATT TAC AAG

CCA GCC ATG TCC TTG TCC GGC CTG TTT GCC AAC GCT GTGCT 3'
||| ||| ||| ||| ||| ||| ||| ||| ||| ||| ||| ||| |||||
GGT CGG TAC AGG AAC AGG CCG GAC AAA CGG TTG CGA C       5'
```

and the ~0.6 kb BamHI-HgiAI fragment of plasmid pCZ101.

X-6354-(R)

16. The process of Claim 1, 2 or 3 for preparing plasmid pCZ1000 which comprises ligating the ~10.2 kb BamHI-XbaI fragment of plasmid pCZ101, a DNA linker having the nucleotide sequence

```
5' CTAGAGGGTATTAATA ATG GAT CAG GAG GAT TAA ATG TTT CCT
   |||||||||||||||| ||| ||| ||| ||| ||| ||| ||| ||| |||
   TCCCATAATTAT TAC CTA GTC CTC CTA ATT TAC AAA GGA

GCT ATG TCC TTG TCC GGC CTG TTT GCC AAC GCT GTGCT 3'
||| ||| ||| ||| ||| ||| ||| ||| ||| ||| ||| |
CGA TAC AGG AAC AGG CCG GAC AAA CGG TTG CGA C        5'
```

and the ~0.6 kb BamHi-HgiAI fragment of plasmid pCZ101.

17. The process of Claim 1, 2 or 3 for preparing plasmid pCZ1000.1 which comprises ligating the ~10.2 kb BamHI-XbaI fragment of plasmid pCZ101, a DNA linker having the nucleotide sequence

```
5' CTAGAGGGTATTAATA ATG GAT CAG TAA ATG TTT CCG GCT ATG
   |||||||||||||||| ||| ||| ||| ||| ||| ||| ||| ||| |||
   TCCCATAATTAT TAC CTA GTC ATT TAC AAA GGC CGA TAC

TCC TTG TCC GGC CTG TTT GCC AAC GCT GTGCT 3'
||| ||| ||| ||| ||| ||| ||| ||| ||| |
AGG AAC AGG CCG GAC AAA CGG TTG CGA C        5'
```

and the ~0.6 kb BamHI-Hgi fragment of plasmid pCZ101.

18. The process of Claim 1, 2 or 3 for preparing plasmid pCZ1060 which comprises ligating the ~10.2 kb BamHI-XbaI fragment of plasmid pCZ101, a DNA linker having the nucleotide sequence

X-6354-(R)

```
5' CTAGAGGGTATTAATA ATG GAT GAT AAG TAA ATG TTC CCA GCC
   |||||||||||||||| ||| ||| ||| ||| ||| ||| ||| ||| |||
   TCCCATAATTAT TAC CTA CTA TTC ATT TAC AAG GGT CGG

ATG TCC TTG TCC GGC CTG TTT GCC AAC GCT GTGCT 3'
||| ||| ||| ||| ||| ||| ||| ||| ||| ||| |
TAC AGG AAC AGG CCG GAC AAA CGG TTG CGA C      5'
```

and the ∿0.6 kb BamHI-HgiAI fragment of plasmid pCZ101.

19. The process of Claim 1, 2 or 3 for preparing plasmid pCZ1120 which comprises ligating the ∿10.2 kb BamHI-XbaI fragment of plasmid pCZ101, a DNA linker having the nucleotide sequence

```
5' CTAGAGGGTATTAATA ATG GAT GAT AAG TAA ATG TTT CCG GCT
   |||||||||||||||| ||| ||| ||| ||| ||| ||| ||| ||| |||
   TCCCATAATTAT TAC CTA CTA TTC ATT TAC AAA GGC CGA

ATG TCC TTG TCC GGC CTG TTT GCC AAC GCT GTGCT 3'
||| ||| ||| ||| ||| ||| ||| ||| ||| ||| |
TAC AGG AAC AGG CCG GAC AAA CGG TTG CGA C      5'
```

and the ∿0.6 kb BamHI-HgiAI fragment of plasmid pCZ101.

20. The process of Claim 1, 2 or 3 for preparing plasmid pCZ105.1 which comprises ligating the EcoRI-ClaI digest of plasmid pNM608, the ∿10.2 kb BamHI-XbaI fragment of plasmid pCZ101, a DNA linker having the nucleotide sequence

```
5' CGACA ATG TTC CCA TTG GAG GAT GAT TAA ATG TTC CCA GCC
   ||| ||| ||| ||| ||| ||| ||| ||| ||| ||| ||| ||| |||
   TGT TAC AAG GGT AAC CTC CTA CTA ATT TAC AAG GGT CGG

ATG TCC TTG TCC GGC CTG TTT GCC AAC GCT GTGCT 3'
||| ||| ||| ||| ||| ||| ||| ||| ||| ||| |||||
TAC AGG AAC AGG CCG GAC AAA CGG TTG CGA C      5'
```

and the ∿0.6 kb BamHI-HgiAI fragment of plasmid pCZ101.

X-6354-(R)

21. The process of Claim 1, 2 or 3 for preparing plasmid pCZ105.2 which comprises ligating the EcoRI-ClaI digest of plasmid pNM608, the ∿10.2 kb BamHI-XbaI fragment of plasmid pCZ101, a DNA linker having the nucleotide sequence

```
5' CGACA ATG TTC CCA TTG GAT GAT GAT TAA ATG TTC CCA GCC
    ||| ||| ||| ||| ||| ||| ||| ||| ||| ||| ||| ||| |||
    TGT TAC AAG GGT AAC CTA CTA CTA ATT TAC AAG GGT CGG

ATG TCC TTG TCC GGC CTG TTT GCC AAC GCT GTGCT 3'
||| ||| ||| ||| ||| ||| ||| ||| ||| ||| |
TAC AGG AAC AGG CCG GAC AAA CGG TTG CGA C        5'
```

and the ∿0.6 kb BamHI-HgiAI fragment of plasmid pCZ101.

22. The process of Claim 1, 2 or 3 for preparing plasmid pCZ106.1 which comprises ligating the EcoRI-ClaI digest of plasmid pNM608, the ∿10.2 kb BamHI-EcoRI fragment of plasmid pCZ101, a DNA linker having the nucleotide sequence

```
5' CGACC ATG GAT CAG GAG TAA ATG TTC CCG GCT ATG TCC
    ||| ||| ||| ||| ||| ||| ||| ||| ||| ||| ||| |||
    TGG TAC CTA GTC CTC ATT TAC AAG GGC CGA TAC AGG

TTG TCC GCC CTC TTT GCC AAC GCT GTGCT 3'
||| ||| ||| ||| ||| ||| ||| ||| |
AAC AGG CGG GAG AAA CGG TTG CGA C        5'
```

and the ∿0.6 kb BamHI-HgiAI fragment of plasmid pCZ101.

-6354-(R)

23. The process of Claim 1, 2 or 3 for preparing plasmid pCZ112.1 which comprises ligating the Eco-RI-ClaI digest of plasmid pNM608, the ∿10.2 kb BamHI-EcoRI fragment of plasmid pCZ101, a DNA linker having the nucleotide sequence

```
5' CGACC ATG GAT GAT AAG TAA ATG TTT CCG GCT ATG TCC
     ||| ||| ||| ||| ||| ||| ||| ||| ||| ||| ||| |||
        TGG TAC CTA CTA TTC ATT TAC AAA GGC CGA TAC AGG

TTG TCC GGC CTC TTT GCC AAC GCT GTGCT 3'
||| ||| ||| ||| ||| ||| ||| ||| |
AAC AGG CCG GAG AAA CGG TTG CGA C        5'
```

and the ∿0.6 kb BamHI-HgiAT fragment of plasmid pCZ101.

24. The process of Claim 1, 2 or 3 for preparing plasmid pCZ112.2 which comprises ligating the EcoRI-ClaI digest of plasmid pNM608, the ∿10.2 kb BamHI-EcoRI fragment of plasmid pCZ101, a DNA linker having the nucleotide sequence

```
5' CGACC ATG GAT GAT AAG GAG TAA ATG TTT CCG GCT ATG
     ||| ||| ||| ||| ||| ||| ||| ||| ||| ||| ||| |||
        TGG TAC CTA CTA TTC CTC ACC TAC AAA GGC CGA TAC

TCC TTG TCC GGC CTC TTT GCC AAC GCT GTGCT 3'
||| ||| ||| ||| ||| ||| ||| ||| ||| |
AGG AAC AGG CCG GAG AAA CGG TTG CGA C        5'
```

and the ∿0.6 kb BamHI-HgiAI fragment of plasmid pCZ101.

25. The process of Claim 1, 2 or 3 for preparing plasmid pCZ1140 which comprises ligating the ∿10.2 kb BamHI-XbaI fragment of plasmid pCZ101, a DNA linker having the nucleotide sequence

0154539

X-6354-(R)

```
5' CTAGAGGGTATTAATA ATG TTC CCA TTG GAG GAT GAT TAA ATG
   ||||||||||||| ||| ||| ||| ||| ||| ||| ||| ||| |||
   TCCCATAATTAT TAC AAG GGT AAC CTC CTA CTA ATT TAC

TTC CCA ACC ATT CCC TTA TCC AGG CTT TTT GAC AAC GCT ATG
||| ||| ||| ||| ||| ||| ||| ||| ||| ||| ||| ||| ||| |||
AAG GGT TGG TAA GGG AAT AGG TCC GAA AAA CTG TTG CGA TAC

CTC CG 3'
||| ||
GAC GC 5'
```

and the Bam HI-FnuDII digest of plasmid pNM575.

26.  A process for preparing plasmid pCZ101 which comprises ligating XbaI-BamHI digest of plasmid pIM-I'-A3 and the XbaI-BamHI digest of plasmid pNM789B.

27.  The process of any of Claims 1 to 25 which comprises the further step of transforming of a host cell with the selectable and autonomously replicating recombinant DNA expression vector.

28.  The process of Claim 27 which comprises transforming a prokaryotic cell.

29.  The process of Claim 28 which comprises transforming an E. coli, Bacillus, or Streptomyces cell.

30.  The process of Claim 29 which comprises transforming E. coli K12 RV308.

31.  A method of producing a functional polypeptide, which comprises

    1)    transforming a cell with a recombinant DNA expression vector produced by the process of any of Claims 1 to 25 and

    2)    culturing the transformed cell under conditions suitable for gene expression.

32. The method of Claim 31 in which the transformed host cell is a prokaryotic cell.

33. The method of Claim 32 in which the transformed prokaryotic cell is E. coli, Bacillus or Streptomyces.

34. The method of Claim 33 in which the transformed cell is E. coli.

35. The method of Claim 34 in which the transformed cell is E. coli K12 RV308.

# FIG. 1

pKEN111

Eco RI   Hpa II
         Xba I
         Pvu II
         Hpa II
         Hpa I

lpp

**101**

TcR

Eco RI

pBR322

Eco RI   Hind III

Pst I            Bam HI

ApR     TcR      Sal I

**102**

Pvu II

↓ Hpa II

950bp Hpa II Fragment

↓ Alu I

462bp Alu I Fragment

↓ Eco RI Linkers
  T4 Ligase

↓ Eco RI

466bp Eco RI
Fragment

Eco RI

Eco RI

Alkaline
Phosphatase

T4 DNA Ligase

5' lpp

lppᴾ

Xba I

Eco RI

Hind III

Bam HI

Sal I

ApR

**103**

TcR

# FIG. 2

**Plasmid 103 (Figure 1)**

Hind III

S1 Nuclease

Bam HI Linkers
T$_4$ DNA Ligase

Bam HI

T$_4$ DNA Ligase

Eco RI

*lpp$_p$*

Xba I
Eco RI
Bam HI

Ap$^R$

**104**

Tc$^s$

Sal I

Eco RI Partial Digestion

S1 Nuclease

Hind III Linkers
T$_4$ DNA Ligase

Hind III

T$_4$ DNA Ligase

Hind III

*lpp$_p$*

Xba I

Eco RI

Ap$^R$

Bam HI

**105**

Sal I

FIG. 3

FIG. 4

# FIG. 5

Plasmid pNM575
(109 in Figure 4)

110

↓ Bam HI

Xba I          Fnu DII

691 bp Bam HI
Fragment

Synthetic
Fragment

↓ Fnu DII

538 bp Bam HI,
Fnu DII Fragment

Plasmid pKEN021
Xba I, Bam HI
(107 in Figure 3)

T₄ DNA Ligase

pNM645

# FIG. 6

Plasmid pNM645
(111 in Figure 5)

Pvu II Partial
Digestion

pBP348

Pst I
Hpa II
Pvu II
Eco RI
Sma I
Pvu II
Pst I
Tc^R
112

Pvu II

494 bp Fragment

Hind III
Ipp^P
Xba I
Pvu II
Ap^R
113
Pvu II
Sal I
3'Ipp
Pvu II

T₄DNA Ligase

Hind III
Ipp^P
Ap^R
114
Xba I
Pvu II
Sma I
Pvu II
Bam HI
3'Ipp
Sal I
Pvu II

pNM685

# FIG. 7

**114** (in Figure 6)

Pvu II Partial Digestion

Xba I

Alkaline Phosphatase

**116**

Xba I ▭▨▨▨▨ Hpa II

Synthetic Fragment

**pPB348**
(112 in Figure 6)

Pvu II

Pst I

135 bp Fragment

Hpa II Partial Digestion

46 bp Fragment

Hind III

Ippρ

Xba I

ApR

**115**

Pvu II

Bovine Growth Hormone

Pvu II

Bam HI

Sal I

**pNM789**

Hind III

Ippρ

Xba I

Hpa II

Pvu II

ApR

**117**

Pvu II

Bam HI

3' Ipp

Pvu II

Sal I

0154539

# FIG. 8

**Plasmid pNM789**
**(117 in Figure 7)**

↓ Pvu II Partial Digestion

↓ Bam HI

↓ Alkaline Phosphatase

**118**

Pvu II          Bam HI

Synthetic Fragment

Hind III

*Ippₚ*

Xba I

Pvu II

↙ Ap^R

**119**

Pvu II

3'Ipp          Bam HI

Sal I

T₄ DNA Ligase

Hind III

*Ippₚ*

Xba I

↙ Ap^R

**120**

Pvu II

Coding Sequence for Bovine Growth Hormone

Pvu II

Bam HI

**pNM789B**

3'Ipp

Sal I

# FIG. 9
## Restriction Site Map of Plasmids pCZ114 and pCZ 105.1

pCZ 114

pCZ 105.1

FIG. 10
**Restriction Site Map of Plasmid pCZ1140**

# FIG. II
## Thymosin Alpha I Gene

Met Ser Asp Ala Ala Val Asp Thr Ser Ser Glu Ile Thr Thr Lys Asp Leu Lys Glu Lys Lys Glu Val Val Glu Glu Ala Glu Asn
(positions 1–28)

Eco RI ... stop stop

AATTCATGTCTGATGCTGCTGTTGATACTTCTTCTGAGATTACTACTAAAGATCTTAAGGAGAAGAAGGAAGTTGTCGAAGAGGCTGAGAACTAATAG

GTACAGACTACGACGACAACTATGAAGAAGACTCTAATGATGATTTCTAGAATTCCTCTTCTTCCTTCAACAGCTTCTCCGACTCTTGATTATCCTAG

T1 T2 T3 T4 T9 T10 T11 T12
T5 T6 T7 T8 T13 T14 T15 T16

Bgl II    Bam HI

# FIG. 12

## Synthesis Procedure for Fragment T₁₅

# FIG. 13
## Construction Route for Plasmid pTh α1

# FIG. 14
## Proinsulin Synthetic Gene

|  | 1 |  |  |  |  |  |  |  |  | 10 |  |  |  |  |  |  |  | 20 |
|---|---|---|---|---|---|---|---|---|---|---|---|---|---|---|---|---|---|---|
| met | phe | val | asn | gln | his | leu | cys | gly | ser | his | leu | val | glu | ala | leu | tyr | leu | val | cys | gly |

```
    ┌────H1────┐ ┌────────H2────────┐ ┌────────H3────────┐ ┌────────H4────────┐ ┌──────B1──────┐ ┌──────B2──────┐
AATTC ATG TTC GTC AAT CAG CAC CTT TGT GGT TCT CAC CTC GTT GAA GCT TTG TAC CTT GTT TGC GGT
    G TAC AAG CAG TTA GTC GTG GAA ACA CCA AGA GTG GAG CAA CTT CGA AAC ATG GAA CAA ACG CCA
    └──────H5──────┘ └────────H6────────┘ └────────H7────────┘ └──────H8──────┘ └──────B6──────┘
```

| glu | arg | gly | phe | phe | tyr | thr | pro | lys | thr | arg | arg | glu | ala | glu | asp | leu | gln | val | gly | gln | val | glu |
|---|---|---|---|---|---|---|---|---|---|---|---|---|---|---|---|---|---|---|---|---|---|---|

```
┌────────B3────────┐ ┌────────B4────────┐ ┌────B5'────┐
GAA CGT GGT TTC TTC TAC ACT CCT AAG ACT CGC CGG GAG GCA GAG GAC CTG CAG GTG GGG CAG GTG GAG
CTT GCA CCA AAG AAG ATG TGA GGA TTC TGA GCG GCC CTC CGT CTC CTG GAC GTC CAC CCC GTC CAC CTC
└──B7──┘ └────B8────┘ └────B9────┘ └─B10'─┘
```

| leu | gly | gly | gly | pro | gly | ala | gly | ser | leu | gln | pro | leu | ala | leu | glu | gly | ser | leu | gln | lys | arg | gly |
|---|---|---|---|---|---|---|---|---|---|---|---|---|---|---|---|---|---|---|---|---|---|---|

```
CTG GGC GGG GGC CCT GGT GCA GGC AGC CTG CAG CCC TTG GCC CTG GAG GGG TCC CTG CAG AAG CGT GGC
GAC CCG CCC CCG GGA CCA CGT CCG TCG GAC GTC GGG AAC CGG GAC CTC CCC AGG GAC GTC TTC GCA CCG
```

| ile | val | glu | gln | cys | cys | thr | ser | ile | cys | ser | leu | tyr | gln | leu | glu | asn | tyr | cys | asn |
|---|---|---|---|---|---|---|---|---|---|---|---|---|---|---|---|---|---|---|---|

```
ATT GTG GAA CAA TGC TGT ACC AGC ATC TGC TCC CTC TAC CAG CTG GAG AAC TAC TGC AAC TAG ACGCAGC
TAA CAC CTT GTT ACG ACA TGG TCG TAG ACG AGG GAG ATG GTC GAC CTC TTG ATG ACG TTG ATC TGCGTCG

CCGCAGGCAGCCCCACACCCGCCGCCTCCTGCACCGAGAGAGATGGAATAAAGCCCTTGAACCAGC · poly A · CCG
GGCGTCCGTCGGGGTGTGGGCGGCGGAGGACGTGGCTCTCTCTACCTTATTTCGGGAACTTGGTCG · poly T · GGCCTAG
```

Amino acids 1-30 are the B chain; 31-65 are the C chain; and 66-86 are the A chain.

14 / 16

0154539

# FIG. 15
## Construction of Plasmid pHI 7△4△1

# FIG.16
## Construction of Plasmid pHI104

mRNA ——— (A)ₙ

Reverse transcriptase + dNTP's
+ BamHI linker
5'CCGGATCCGG(t)ₙ3'

——— (A)ₙ 3'
——— (T)ₙ GGCCTAGGCC 5'

Denature RNA/DNA hybrid

Klenow Pol I

BamHI
——— (A)ₙCCGGATCCGG 3'
——— (T)ₙGGCCTAGGCC 5'

SI Nuclease

Terminal transferase + dCTP

——— (A)ₙCCGGATCCGGCCCCC
CCCCC ——— (T)ₙGGCCTAGGCG

EcoRI
BamHI
PstI
AprR    pBR322    TcR

PstI
Terminal transferase
+ dGTP

GGG GGG

Anneal
Transform E. coli
Select TcR
Screen

EcoRI    BamHI
PstI
HpaII
PstI    pHI104    TcR
Human Insulin
cDNA
BamHI
PstI
ApS